# EUROPEAN PATENT APPLICATION

(11) **EP 2 387 994 A1**
(43) Date of publication of application: **23.11.2011**
(21) Application number: 11001815.7
(22) Date of filing: 29.01.2007
(51) Int. Cl.: A61K 9/22

(54) **Drug delivery systems comprising weakly basic drugs and organic acids**

(30) Priority: 27.01.2006 US 762766 P
(62) Divisional of application: 07717476.1
(71) Applicant: Eurand, Inc., Vandalia, OH 45377 (US)
(72) Inventor: Vankatesh, Gopi M., Vandalia, OH 45377 (US)
(74) Representative: Kremer, Simon Mark

(57) **Abstract**

A pharmaceutical dosage form such as a capsule, a conventional or orally disintegrating tablet capable of delivering a nitrogen (N)-containing therapeutic agent having a pKa in the range of from about 5 to 14 into the body in a sustained-released fashion, in order to be suitable for a twice- or once-daily dosing regimen, comprises at least one organic acid, which solubilizes the therapeutic agent the drug prior to releasing it into the hostile intestinal environment wherein said weakly basic drug is practically insoluble. The unit dosage form is composed of a multitude of timed, pulsatile-release beads and is designed in such a way that said weakly basic drug and said organic acid do not come into close contact during processing and/or storage for in-situ formation of acid addition compounds while ensuring that the acid is not depleted prior to completion of the drug release.

## Description

### CROSS-REFERENCE TO RELATED APPLICATION

This application claims the benefit of U.S. Provisional Application No. 60/762,766 filed January 27, 2006, the contents of which are hereby incorporated by reference.

### TECHNICAL FIELD

The present invention relates to the development of modified-release dosage forms comprising one or more timed, pulsatile-release bead populations of a weakly basic, nitrogen (N)-containing therapeutic agent having a pKa in the range of from about 5 to 14 and a solubility of not more than 200 µg/mL at a pH of 6.8, and one or more pharmaceutically acceptable organic acids. The dosage form exhibits comparable release profiles of both the active and the organic acid after a predetermined delay (lag time) when dissolution tested by United States Pharmacopoeia (USP) dissolution methodology using a two-stage dissolution medium (first 2 hours in 0.1N HCl followed by testing in a buffer at pH 6.8). In accordance with another aspect of the invention, oral drug delivery systems to target PK (pharmacoldnetics, i.e., plasma concentration-time) profiles suitable for a once- or twice-daily dosing regimen in patients in need of a medication are disclosed.

### BACKGROUND OF THE INVENTION

Many therapeutic agents are most effective when made available at constant rates at or near the absorption sites. The absorption of therapeutic agents thus made available generally results in desired plasma concentrations leading to maximum efficacy, and minimum toxic side effects. Much effort has been devoted to developing sophisticated drug delivery systems such as osmotic devices for oral application. However, there are instances where maintaining a constant blood level of a drug is not desirable. For example, a major objective of chronotherapy for cardiovascular diseases is to deliver the drug in higher concentrations during the time of greatest need, e.g., the early morning hours, and in lesser concentrations when the need is less, e.g., during the late evening and early sleep hours. In addition to a properly designed drug delivery system, the time of administration is equally important. The unique pharmacokinetic profile needed can be calculated using computer simulation and modeling techniques based on the knowledge of pharmacokinetic parameters, solubility, absorption along the gastrointestinal tract and elimination half-life.

While the orally administered pharmaceutical dosage form passes through the human digestive tract, the drug should be released from the dosage form and be available in solution form at or near the site for absorption from the gastrointestinal (GI) tract to occur. The rate at which the drug goes into solution and is released from a dosage form is important to the kinetics of drug absorption. The dosage form and hence the active ingredient is subjected to varying pHs during the transit, i.e., pH varying from about 1.2 (stomach pH during fasting but may vary between 1.2 and 4.0 upon consumption of food) to about 7.4 (bile pH: 7.0-7.4 and intestinal pH: 5 to 7). Moreover, transit time of a dosage form in individual parts of the digestive tract may vary significantly depending on its size and prevailing local conditions. Other factors that influence drug absorption include physicochemical properties of the drug substance itself such as pKa, solubility, crystalline energy, and specific surface area. The prevailing local conditions that play an important role include properties of luminal contents (pH, surface tension, volume, agitation and buffer capacity) and changes following the ingestion of food. Consequently, it is often difficult to achieve drug release at constant rates.

Basic and acidic drugs exhibit pH-dependent solubility profiles varying by more than 2 orders of magnitude in the physiological pH range. The most difficult candidates to work with are weakly basic pharmaceutically actives, which are practically insoluble at a pH >6 and require high doses to be therapeutically effective. Upon entering into the intestinal region, part of the drug released from the dosage form may precipitate in the hostile pH environment unless the rate of absorption is faster than the rate of drug release. Alternatively, the drug may remain in the supersaturated solution state facilitated by the presence of bile salts and lecithin in the gut. A supersaturation well over an order of magnitude higher than the aqueous solubility has been evident in the prior art. In the event of precipitation, there is evidence of redissolution for absorption at a slower phase.

Functional polymer membranes comprising suitable combinations of synthetic polymers such as water-soluble (e.g., Povidone), water-insoluble (e.g., ethyl cellulose insoluble at physiological pHs), gastrosoluble (e.g., Eudragit EPO) or enterosoluble (e.g., gastric-resistant hypromellose phthalate) polymers, have been applied on tablet or pellet cores comprising the active and one or more solubilizers to achieve drug release at constant rates with limited success. Development of pharmaceutical compositions of actives highly water soluble at acidic or basic pHs using pharmaceutically acceptable buffer acids, buffer acid salts, and mixtures thereof, to provide drug release at substantially constant rates have been described. Organic acids have been used to improve bioavailability, to reduce inter and intra-subject variability, and to minimize food effect in weakly basic pharmaceutical actives. Multi-particulate dosage forms comprising weakly basic drugs to provide extended-release profiles are also described in the literature. These dosage forms typically are obtained by granulating or layering the drug with one or more organic acids and coating with a combination of water-insoluble and water-soluble or enteric polymers.

Although the drug release in these disclosures could be extended moderately, they suffered from two disadvantages, viz., failure to maintain adequate plasma profile to achieve a once-daily dosing regimen and partial to complete *in situ* formation of the salt form, thus creating a new chemical entity. Even when the organic acid containing cores were coated with a sustained-release polymer membrane, the delivery system failed to prolong the release of the acid for continued dissolution and resulting absorption of the active to provide adequate plasma levels at 24 hrs following oral ingestion. Furthermore, many weakly basic drugs are known to form salts in the presence of organic acids, especially when dissolved in common solvents for drug layering or during granulation. Even in dosage forms wherein the organic acid and the drug layers are separated by a sustained release (SR) membrane, the drug layering formulation contains an organic acid. Consequently, the active in the finished dosage exists in the partially or fully neutralized salt form. This is not an acceptable situation from regulatory considerations. The regulatory agencies may consider these actives as new drug entities. Thus there is an unmet need to develop drug delivery systems comprising weakly basic drugs with a pKa in the range of from about 5 to 14 and requiring high doses and organic acids in an unaltered form to release the actives so as to maintain target plasma concentrations of Cₘₐₓ and Cₘᵢₙ in order to be suitable for once-daily dosing regimens. After extensive investigations, it was surprisingly discovered that this unmet need can be met by preventing the organic acid and the weakly basic active agent from coming into contact with each other to form a salt during processing and/or in the dosage form during storage, prior to dropping into an *in vitro* dissolution medium or prior to oral administration. This could be achieved by applying a dissolution rate-controlling SR membrane between the acid layer on the inert cores and the drug layer applied onto the acid-containing cores to isolate these two components and also an SR and/or a TPR (lag-time coating) membrane on the IR beads in order to synchronize the acid release with that of the drug.

### SUMMARY OF THE INVENTION

The present invention provides pharmaceutical compositions and methods for creating pulsatile delivery systems, which involves preventing a weakly basic, nitrogen (N)-containing therapeutic agent having a pKa in the range of from about 5 to 14 (typically soluble at acidic pHs, but poorly to practically insoluble at neutral and alkaline pHs) and an elimination half-life of about 2 hours or longer, and a pharmaceutically acceptable organic acid from coming into contact to form an acid addition compound. Furthermore, the dosage forms described herein provide target drug-release profiles by solubilizing the drug prior to releasing it into the hostile intestinal environment wherein the drug is practically insoluble, thereby enhancing the probability of achieving acceptable plasma concentration up to 12-24 hour post-dosing in order to be suitable for a twice- or once-daily dosing regimen.

Another embodiment of the invention relates to a multiparticulate pharmaceutical composition comprising one or more coated bead populations containing one or more weakly basic, nitrogen (N)-containing therapeutic agents having a pKa in the range of from about 5 to 14, a solubility of not more than about 200 µg/mL at pH 6.8, and a ratio of optimal highest dose to the solubility at pH 6.8 of at least about 100. For example, if the dosing regimen for an immediate-release (IR) dosage form of a drug with a solubility of 0.05 mg/mL at pH 6.8 is 5 mg twice a day, the optimal highest dose is 10 mg once-daily and the ratio of optimal highest dose (mg) to the solubility (mg/mL) at pH 6.8 would be 200. The multiparticulate composition prepared in accordance with one aspect of the present invention will comprise organic acid-containing cores coated with a barrier membrane (e.g., an SR (sustained-release)), on which a weakly basic therapeutic agent with a pKa in the range of from about 5 to 14, is layered and further coated with an SR membrane and/or a lag-time membrane such that both the organic acid and the weakly basic therapeutic agent exhibit comparable drug-release profiles.

Multiparticulate compositions prepared in accordance with one aspect of the present invention comprise one or more coated bead populations exhibiting similar composite release profiles of both the organic acid and the weakly basic nitrogen (N)-containing therapeutic agent when tested for dissolution using United States Pharmacopoeia Apparatus 1 (baskets @ 100 rpm) or Apparatus 2 (paddles @ 50 rpm) and a two-stage dissolution methodology (testing in 700 mL of 0.1N HCl (hydrochloric acid) for the first 2 hours and thereafter in 900 mL at pH 6.8 obtained by adding 200 mL of a pH modifier). Another embodiment of the invention relates to a multiparticulate pharmaceutical composition comprising one or more coated bead populations exhibiting the acid-release profile which is more particularly slower in comparison to that of the weakly basic active in order to avoid undissolved active being left behind inside the coated beads.

A multiparticulate pharmaceutical composition in accordance with one aspect of the invention comprises coated bead populations of a weakly basic pharmaceutical active with a pKa in the range of from about 5 to 14 comprising:
a) an organic acid-containing core particle (organic acid crystal, pellet, bead and the like);
b) a barrier or sustained-release membrane on the acid-containing core particle comprising a water-insoluble polymer or a water-insoluble polymer in combination with a pore-forming water-soluble or enteric polymer;
c) a weakly basic drug layered on the barrier-coated acid-containing core particle and optionally provided with a protective seal-coat to form an immediate-release (IR) bead;
d) if providing SR beads, an SR coating membrane on the IR bead comprising a water-insoluble polymer or a water-insoluble polymer in combination with a water-soluble polymer forming an SR bead; and / or
e) if providing timed, pulsatile-release (TPR) beads, a lag-time coating membrane on the SR-coated bead or directly on the IR bead comprising a combination of a water-insoluble and enteric polymers to form a TPR bead.

The compositions in accordance with particular aspects of the invention typically exhibit desired or target release profiles of both the active and organic acid following a pre-determined lag-time of at least 2 hours when tested for drug and/or organic acid release using the 2-stage dissolution methodology described above.

A pharmaceutical composition of a weakly basic, nitrogen (N)-containing therapeutic agent having a pKa in the range of from about 5 to 14, a solubility of not more than about 200 µg/mL at pH 6.8, and a ratio of optimal highest dose to solubility at pH 6.8 of not less than about 100 may be prepared by filling the corresponding bead populations into a hard gelatin capsule or compressing into a conventional tablet or in the ODT (orally disintegrating tablet) form in accordance with certain embodiments of the present invention.

A pharmaceutical composition of a weakly basic therapeutic agent in the ODT form prepared in accordance with another embodiment of the present invention disintegrates on contact with saliva in the buccal cavity within about 60 seconds forming a smooth, easy-to-swallow suspension (no gritty or chalky aftertaste). The pharmaceutical composition of a weakly basic pharmaceutical active in the ODT form which may comprise one or more coated bead populations with an average particle size of not more than about 400 µm, such as taste-masked microcapsules comprising drug-containing cores (crystals, granules, pellets, beads and the like), SR bead and timed, pulsatile-release (TPR) bead populations comprising SR coated acid-containing cores. Taste-masking may be achieved by any of the well-known prior art disclosures. The ODT may also include rapidly-dispersing microgranules with an average particle size of not more than about 400 µm, or in some embodiments not more than about 300 µm, comprising a disintegrant (e.g., Crospovidone, crosslinked polyvinylpyrrolidone) and a sugar alcohol (e.g., mannitol), a saccharide (e.g., lactose) or a combination thereof, each having an average particle size of not more than about 30 µm, and, optionally, pharmaceutically acceptable excipients typically used in ODT formulations, viz., flavors, a sweetener, coloring agents, and additional disintegrant.

The ODT in accordance with one embodiment exhibits the following properties:
1) disintegrates on contact with saliva in the oral cavity in about 60 seconds forming a smooth, easy-to-swallow suspension comprising taste-masked and/or coated particles (SR and/or TPR beads);
2) taste-masked particles, if present, provide rapid, substantially-complete release of the dose upon entry into the stomach (e.g., typically greater than about 75% in about 60 minutes);
3) coated particles (SR and/or TPR beads) provide prolonged release of the active for continued absorption along the GI tract.

The ODT in accordance with one embodiment comprising taste-masked microparticles demonstrating effective taste-masking by releasing not more than 10% in about 3 minutes (the longest typical residence time anticipated for the ODT in the buccal cavity) when dissolution tested in simulated saliva fluid (pH∼ 6.8) while releasing not less than about 50% of the dose in about 30 minutes when dissolution tested in 0.1N HCl.

In accordance with certain embodiments, the rapidly-dispersing microgranules and coated beads (taste-masked IR, SR and/or TPR beads) of one or more weakly basic actives may be present in the weight ratio of about 6:1 to 1:1, more particularly from about 4:1 to 2:1, to achieve a smooth mouth feel. In accordance with certain other embodiments, the coated beads (taste-masked IR, SR and/or TPR beads) of one or more weakly basic actives may be coated with a compressible coating (e.g., fluid-bed coating with a plasticized aqueous dispersion of ethylcellulose) in order to minimize membrane fracture during compression with rapidly-dispersing microgranules.

A pharmaceutical composition of a weakly basic pharmaceutical active in the conventional tablet form in accordance with another embodiment of the present invention, may comprise one or more bead populations, such as IR beads (crystals, granules, pellets, beads and the like), and SR beads and/or TPR beads comprising SR coated acid-containing cores. The pharmaceutical composition of a weakly basic pharmaceutical active in the conventional tablet form disintegrates into constituent beads (taste-masked particles, coated SR beads and/or TPR beads) upon oral ingestion in about 10 minutes. The conventional tablet may also include pharmaceutically acceptable excipients typically used in disintegrating tablet formulations such as compressible diluents, fillers, coloring agents, and optionally a lubricant.

The conventional tablet prepared in accordance with one embodiment exhibits the following properties:
1) disintegrates upon oral ingestion in about 10 minutes into IR particles and/or coated particles (SR and/or TPR beads);
2) IR particles, if present, provide rapid, substantially-complete release (e.g., greater than about 95%) of the dose within about 60 minutes, more particularly within about 30 minutes upon entry into the stomach;
3) SR and/or TPR beads provide prolonged release of the active for continued absorption along the gastrointestinal (GI) tract.

Another embodiment of the invention relates to a multiparticulate pharmaceutical composition comprising one or more coated bead populations comprising one or more weakly basic therapeutic agents having an elimination half-life of about 2 hours or longer, wherein the active is layered onto SR coated organic acid-containing cores. The pulsatile delivery system developed in accordance with this aspect of the present invention may comprise IR bead, SR bead and timed, pulsatile-release (TPR) bead populations. The SR coated organic acid-containing cores are typically prepared by layering an organic acid (e.g., fumaric acid) onto inert particles (e.g., sugar spheres) from a polymeric binder solution and coated with a water-insoluble polymer (e.g., ethylcellulose, with a viscosity of about 10 cps) alone or in combination with a water-soluble polymer (e.g., polyvinylpyrrolidone, Povidone K-25 or polyethylene glycol, PEG 400) or an enteric polymer (e.g., hypromellose phthalate, HPMCP or HP-55). The IR bead population comprising SR coated acid-containing cores are prepared by drug layering onto SR coated acid-containing cores from a polymeric binder solution and providing a protective seal coat of Opadry Clear or Pharmacoat™ 603. The SR and TPR bead populations are prepared by coating IR beads with a water-insoluble polymer (e.g., ethylcellulose) alone or in combination with a water-soluble polymer (e.g., PVP K-25 or PEG 400) or an enteric polymer (e.g., hypromellose phthalate, HPMCP or HP-55). The IR bead population comprising SR coated acid-containing cores are prepared by drug layering onto SR coated acid-containing cores from a polymeric binder solution and providing a protective seal coat of Opadry Clear. The SR and TPR bead populations are prepared by coating IR beads with a water-insoluble polymer (e.g., ethylcellulose) alone or in combination with a water-soluble polymer (e.g., PVP K-25 or PEG 400). In accordance with one aspect of the invention each SR or TPR bead population releases both the drug and the acid at comparable rates, as rapid-release or sustained-release profiles after a pre-determined lag-time (for example, a lag-time of up to10 hours) upon oral administration. IR beads, if included in the dosage form (capsule or conventional tablet or orally disintegrating tablet), may comprise the drug layered directly onto inert cores and coated with a protective seal coat or a taste-masking membrane, which being part of the total dose, provides for rapid absorption (a bolus dose) upon oral administration.

A method of manufacturing a multiparticulate pharmaceutical composition wherein a delivery system developed in accordance with certain embodiments of the present invention comprises one or more weakly basic active pharmaceutical ingredients in sufficient quantities to be administered orally to a patient at prescribed twice- or once-daily dosing regimen to provide therapeutic efficacy is also provided.

The method of manufacturing a multiparticulate pharmaceutical composition in accordance with particular embodiments includes layering of a pharmaceutically acceptable organic acid such as fumaric acid from a polymeric binder solution onto inert particles selected from the group consisting of sugar spheres and cellulose spheres. Fluid bed or pan coating may be used to apply the organic acid and polymeric binder solution. In accordance with other embodiments, the core particles may be crystals with a desired particle size distribution, microgranules, pellets or beads containing one or more organic acid(s). In accordance with certain embodiments, the microgranules, extruded-spheronized pellets or compressed microtablets comprising one or more organic acids, a polymeric binder, which imparts resilient characteristics to dried microgranules, hydrophilic fillers/diluents, and optionally a flavor, a sweetener and/or a disintegrant These organic acid-containing particles are barrier coated with an SR (sustained release) polymer membrane comprising a water-insoluble polymer (e.g., ethylcellulose with an average viscosity of 10 cps) alone or in combination with a water-soluble polymer (e.g., polyvinyl pyrrolidone or polyethylene glycol) or an enteric polymer (e.g., hypromellose phthalate (HPMCP or HP-55)). The water-insoluble and water-soluble or enteric polymers may be present at a weight ratio of from about 95:5 to about 50:50, more particularly from about 90:10 to 60:40 and the membrane thickness may vary from about 3% to 50%, more particularly from about 5% to 30% by weight in accordance with particular embodiments.

In accordance with particular embodiments, one or more weakly basic drug(s) are applied onto barrier-coated acid-containing particles from a polymeric binder solution and also, a protective seal coat with a hydrophilic polymer (e.g., Pharmacoat™ 603 or Opadry^{®} Clear) is applied on drug-layered beads to produce IR beads. The organic acid or drug load depends on the physicochemical as well as the pharmacological properties of the weakly basic actives chosen for development, and the drug and the organic acid may be present at a weight ratio of from about 5:1 to 1:10, or more particularly from about 3:1 to 1:3 depending on whether organic acid crystals or organic acid-containing cores are used in accordance with certain embodiments.

In accordance with certain embodiments of the present invention, the IR beads comprising barrier-coated acid-containing cores are barrier coated with an SR polymer membrane comprising a water-insoluble polymer (e.g., ethylcellulose with an average viscosity of 10 cps) alone or in combination with a water-soluble polymer (e.g., polyvinyl pyrrolidone or polyethylene glycol). The water-insoluble and water-soluble polymers may be present at a weight ratio of from about 95:5 to about 50:50, more particularly from about 90:10 to 60:40 and the membrane thickness may vary from about 3% to 50%, more particularly from about 5% to 30% by weight in accordance with particular embodiments.

In accordance with other embodiments of the present invention, the SR beads comprising drug-layered beads are coated with a lag-time membrane comprising a combination of a water-insoluble polymer (e.g., ethylcellulose with an average viscosity of 10 cps) and an enteric polymer (e.g., hypromellose phthalate (HPMCP or HP-55)) to produce TPR beads. In accordance with certain other embodiments, the water-insoluble and enteric polymers may be present at a weight ratio of from about 9:1 to about 1:4, more particularly from about 3:1 to 1:1, and the membrane thickness may vary from about 5% to 60%, more particularly from about 15% to 50% by weight in accordance with particular embodiments.

The functional polymeric systems being applied from aqueous or solvent-based compositions typically contain plasticizers at suitable concentrations. The finished dosage form may be a modified-release (MR) capsule, a standard (conventional) tablet or an orally disintegrating tablet (ODT) comprising a coated spherical bead population containing the active substance alone or a combination of two or more coated bead populations to provide target plasma concentrations suitable for a once-daily dosing regimen. For example, a once-daily dosage form of an active with an elimination half-life of about 7 hours may contain a mixture of an IR bead population which allows immediate release, a second, TPR bead population with a shorter lag-time (about 3-4 hours), which allows a delayed, rapid -release and a third TPR bead population with a longer lag-time (about 7-8 hours), which allows typically a delayed, sustained-release profile over about 8-12 hours, to maintain acceptable plasma concentrations at 12-24 hrs, thus enhancing safety, therapeutic efficacy and patient compliance while reducing cost of treatment. Alternatively, the finished dosage form may comprise an IR bead population and a second, TPR bead population with a lag-time of about 7-8 hours followed by a sustained-release profile over 10-12 hours. The achievable lag time depends on the composition and thickness of the barrier coating, as well as the composition and thickness of the lag-time coating. Specific factors that can affect achieving optimal twice- or once-daily dosage forms include, but are not limited to, the therapeutic agent's pKa (and its solubility above a pH of 6.0), elimination half-life, and solubility enhancement in an aqueous solution of an organic acid selected from the group consisting of aspartic acid, citric acid, fumaric acid, maleic acid, oxalic acid, succinic acid, tartaric acid, and the like.

In accordance with certain embodiments of the present invention, a method of manufacturing a multiparticulate composition comprising a weakly, nitrogen (N)-containing therapeutic agent having a pKa in the range of from about 5 to 14 and a solubility of not more than 200 µg/mL at a pH of 6.8, is also provided. The method may comprise the steps of:
a) preparing core particles (crystals with a particle size distribution of 20-500 µm, more particularly of 100-300 µm, beads or pellets) of one or more pharmaceutically acceptable organic acids;
b) coating these acid-containing cores with a water-insoluble polymer or a water-insoluble polymer in combination with a water-soluble or enteric polymer in order to program the release of the acid for a weight gain of from about 3% to 50%;
c) layering said weakly basic nitrogen (N)-containing therapeutic agent from a polymeric binder solution and applying a protective seal-coat onto the drug-layered beads to produce IR beads;
d) applying a barrier (sustained-release) coating of a water-insoluble polymer or a water-insoluble polymer in combination with a water-soluble polymer for a weight gain of from about 3% to 30% to produce SR beads;
e) applying a lag-time (time-delay) coating of a combination of water-insoluble and enteric polymers at a weight ratio of from about 10:1 1 to 1:4 for a weight gain of from about 10% to 60% by weight of the coated bead to produce TPR beads; and
f) filling into hard gelatin capsules or compressing into conventional tablets / orally disintegrating tablets (ODTs) after blending with pharmaceutically acceptable excipients and one or more bead populations (e.g., a combination of IR beads, SR beads and/or TPR beads at a desired ratio).

The composition comprising one or more bead populations (e.g., a combination of IR and TPR bead populations) may exhibit the following properties:
a) the composition disintegrates on contact with saliva in the oral cavity forming a smooth, easy-to-swallow suspension (if in the ODT form) or disintegrates within about 10 minutes upon oral ingestion (if in the conventional tablet or capsule form);
b) the IR beads, taste-masked or not, rapidly release of the dose upon entry into the stomach (e.g., typically greater than about 50%, more particularly greater than about 75%, in about 60 minutes);
c) the SR or TPR beads release the drug over a period of about 4 to 20 hours in synchronization with that of the organic acid after a predetermined delay (e.g., up to about 10 hours) following oral administration;
d) the composite drug-release profile of the composition is similar to target *in vitro* drug-release/*in vivo* plasma concentration profile in order to be suitable for a twice- or once-daily dosing regimen.

These and other embodiments, advantages and features of the present invention become clear when detailed descriptions and examples are provided in subsequent sections.

### BRIEF DESCRIPTION OF THE DRAWINGS

FIG. 1 illustrates pH-solubility profiles for (a) Ondansetron hydrochloride, (b) Carvedilol, (c) Dipyridamole, and (d) Clonazepam;

FIG. 2 illustrates a cross-section of an SR coated organic acid-containing core in accordance with one aspect of the invention;

FIG. 3 illustrates a cross-section of a TPR bead comprising an SR coated organic acid-containing core in accordance with a particular aspect of the invention;

FIG. 4 illustrates the release of fumaric acid from SR-coated acid crystals coated at different ratios of EC-10/PEG of Example 1;

FIG. 5 illustrates the release profiles of dipyridamole from TPR beads of Example 2E;

FIG. 6 illustrates the release profiles of carvedilol TPR beads of Example 5 versus Comparative Example 4B;

FIG. 7 illustrates the release profiles of ondansetron hydrochloride from TPR beads on stability (Example 5);

FIG. 8 illustrates the release profiles of ondansetron hydrochloride from TPR beads coated at different ratios of EC-10/HP-55/TEC at 50% by weight of Example 5; and

FIG. 9 illustrates the plasma concentration - time profiles of ondansetron HCl Test Formulations (QD) and Zofran 8 mg (BID) of Example 7.

### DETAILED DESCRIPTION OF THE INVENTION

All documents cited are, in relevant part, incorporated herein by reference; the citation of any document is not to be construed as an admission that it is prior art with respect to the present invention.

As used herein, as well as in specific examples thereof, the term "weakly basic pharmaceutical active" includes the base, pharmaceutically acceptable salts, polymorphs, stereoisomers and mixtures thereof. This term, which is more fully defined in a subsequent section, refers to a nitrogen (N)-containing therapeutic agent having a pKa in the range of from about 5 to 14, more particularly to an agent having a solubility of not more than 200µg/mL at a pH of 6.8.

As used herein, the term "immediate release" refers to release of greater than or equal to about 50%, especially if taste-masked for incorporation into an orally disintegrating tablet dosage form, preferably greater than about 75%, more preferably greater than about 90%, and in accordance with certain embodiments greater than about 95% of the active within about 2 hours, more particularly within about one hour following administration of the dosage form. The term can also refer to the release of the active from a timed, pulsatile release dosage form characterized by an immediate release pulse after the designed lag time. The term "lag-time" refers to a time period wherein less than about 10%, more particularly substantially none, of the dose (drug) is released, and a lag-time of from at least about 2 to 10 hours is achieved by coating typically with a combination of water-insoluble and enteric polymers (e.g., ethylcellulose and hypromellose phthalate).

Unless indicated otherwise, all percentages and ratios are calculated by weight based on the total composition.

An aqueous or a pharmaceutically acceptable solvent medium may be used for preparing organic acid-containing core particles for drug layering, viz., acid-containing beads by layering an acid onto inert cores (e.g., sugar spheres) or IR beads by drug-layering onto acid-containing cores or directly onto sugar spheres from an appropriate polymer binder solution in fluid-bed equipment. Also, an aqueous dispersion of functional polymers, which are available as dispersions or a solvent system may be used for dissolving functional polymers for coating acid-containing beads, IR beads or SR beads.

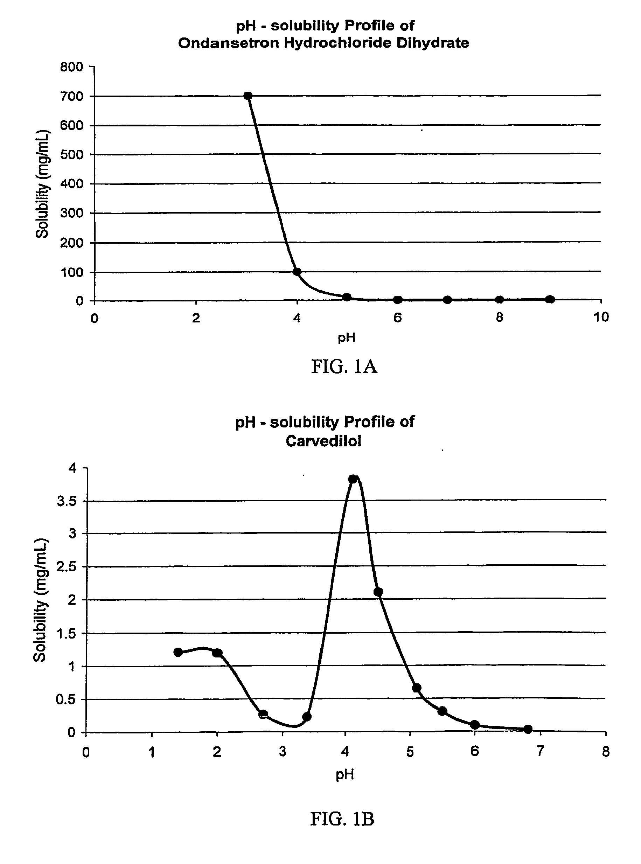

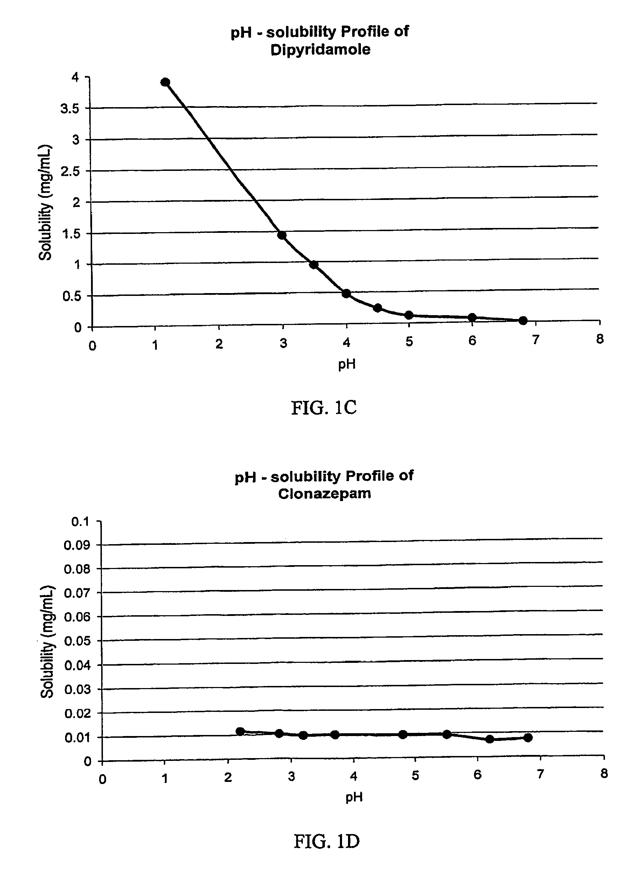

Many active pharmaceutical ingredients (API) are weakly basic in the sense that these actives are freely to moderately soluble at acidic pHs, but are poorly to practically insoluble at neutral and alkaline pHs. Their pKa values are in the range of about 5 to 14. The pH-dependent solubility data for typical weakly basic actives are presented in Fig. 1. For example, dipyridamole's solubility in 0.1N HCl (hydrochloric acid) is about 1 mg/mL, while at pH 6.8 the solubility is only 30 µg/mL. Although carvedilol's solubility is similarly pH-dependent and varying, it is not obvious from Fig. 1 as it rapidly undergoes *in situ* salt formation with the buffering agent such as citric, acetic, and hydrochloric acids and consequently, the observed solubility is that of the salt formed *in-situ.*

Table 1 lists the solubility enhancement of weakly basic actives in organic acid buffers. Three distinct groups can be identified. Group A actives, as represented by ondansetron hydrochloride, exhibit a dramatic increase in solubility of the weakly basic active in a buffer with a trace of fumaric acid. For example, ondansetron's solubility of about 26 mg/mL in the buffer containing only 0.05 mg/mL of fumaric acid remains unchanged upon increasing the concentration of fumaric acid in the buffer up to 5 mg/mL. In Group B, represented by dipyridamole, carvedilol and lamotrigine, the weakly basic drug's solubility increases with increasing concentration of the acid. In Group C, represented by clonazepam, the organic acid has very limited impact, i.e., the solubility enhancement amounts typically to less than about 3-fold. For example, clonazepam's solubilities are about 11.6 and 6.9 µg/mL in buffers at pH 2.3 and 6.8 containing a higher and lower concentration of fumaric acid, respectively.

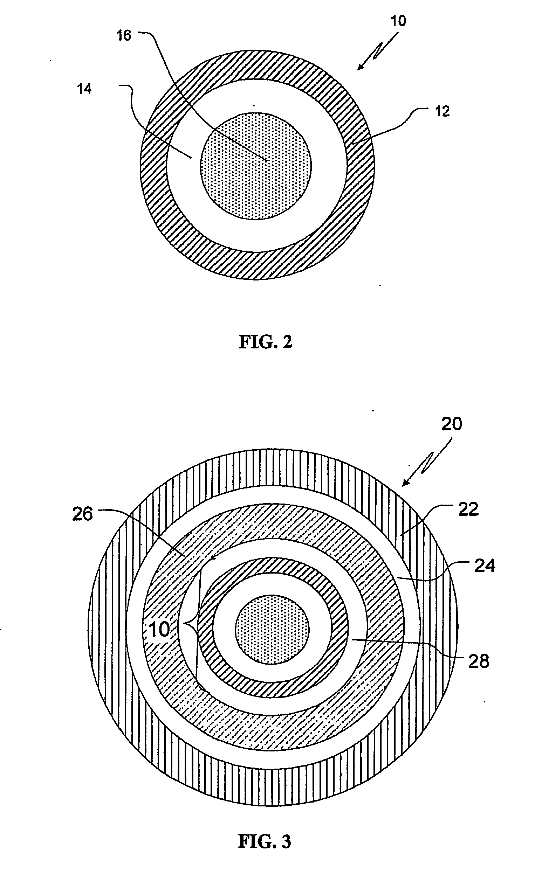

Specific embodiments of the invention will be described in further detail with reference to the accompanying Figures 2 and 3. In Fig. 2, an SR-coated core 10 comprising an SR coating 12 applied on an organic acid-containing core comprising a layer of a pharmaceutically acceptable organic acid in a binder 14 coated on an inert particle core *16.* The inert particle core *16*, organic acid-coating layer *14* and a dissolution rate controlling SR layer *12* make up the SR-coated organic acid-containing core *10.* In Fig. 3, a representative TPR bead is illustrated. The TPR bead *20* comprises a lag-time coating *22* applied on a primary SR layer *24*, a protective seal-coat *26* and a weakly basic drug layer *28* applied on an SR-coated acid-containing core *10.* The weakly basic drug is typically applied from a polymeric binder solution. The SR coating sustains the drug release while the lag-time coating provides the lag-time (a time period exhibiting less than about 10%, more particularly substantially none, of the dose released). Thus the lag-time coating *22*, outer SR coating on the IR beads *24*, and inner SR coating *12* on the acid-containing core together control the release properties of both the drug and acid from the TPR beads.

**Table 1: Solubilities of Weakly Basic Drugs in Organic Acids**

| **Concentration of Fumaric Acid (mg/mL)** | **Start pH** | **End pH** | **Solubility of Ondansetron Hydrochloride (mg/mL)** | **Start pH** | **Solubility of Dipyridamole (mg/mL)** |
|---|---|---|---|---|---|
| 5 | 2.13 | 2.01 | 26.9 | 2.98 | 6.24 |
| 2.5 | 2.26 | 2.14 | 27.0 | 3.42 | 1.80 |
| 1 | 2.48 | 2.40 | 26.1 | 3.68 | 0.93 |
| 0.25 | 2.79 | 2.75 | 26.2 | 3.88 | 0.65 |
| 0.05 | 3.19 | 3.49 | 26.0 | 4.33 | 0.27 |
| 0.01 | 3.64 | 4.05 | 26.1 | 4.71 | 0.13 |
| 0.0025 | 4.15 | 4.33 | 26.1 | 6.28 | 0.006 |
| | | | | | |

| **Solubility (mg/mL) of Carvedilol in Tartaric Acid** | | **Solubility (mg/mL) of Lamotrigine in Tartaric Acid** | | **Solubility (mg/mL) of Clonazepam in Fumaric Acid** | |
|---|---|---|---|---|---|
| **pH of Buffer** | **(mg/mL)** | **pH of Buffer** | **(mg/mL)** | **pH of Buffer** | **(mg/mL)** |
| 2.12 | 2.51 | 2.43 | 4.48 | 2.3 | 0.0116 |
| 2.28 | 1.36 | 3.33 | 1.77 | 2.8 | 0.0103 |
| 2.54 | 0.731 | 4.36 | 1.61 | 3.2 | 0.0096 |
| 2.94 | 0.508 | 4.97 | 0.488 | 3.7 | 0.0098 |
| 3.64 | 0.121 | 5.66 | 0.226 | 4.8 | 0.0095 |
| 5.46 | 0.105 | 5.85 | 0.197 | 5.5 | 0.0093 |
| 5.90 | 0.028 | 6.50 | 0.161 | 6.2 | 0.0072 |
| | | | | 6.8 | 0.0069 |

The novelty/utility of the formulations developed in accordance with certain embodiments of the present invention is disclosed using ondansetron hydrochloride, lamotrigine, dipyridamole, and carvedilol as examples of weakly basic, nitrogen (N)-containing therapeutic agents having a pKa in the range of from about 5 to 14 and a solubility of not more than about 200 µg/mL at pH 6.8. Ondansetron hydrochloride, a selective blocking agent of the serotonin 5-HT₃ receptor, is an anti-emetic and anti-vomiting agent. It is fairly soluble at acidic pHs while practically insoluble at a pH of 6.8. Dipyridamole, an antiplatelet agent, is chemically a dipiperidine derivative. It is soluble in dilute acids and practically insoluble in water, neutral and alkaline buffers.

Carvedilol, a beta-blocker with additional vasodilating, antiproliferative properties, is indicated for the treatment of high tension (HF), coronary artery disease and congestive heart failure. The current commercial formulation of carvedilol is immediate release, and is administered twice daily. The immediate dosage form is rapidly and extensively absorbed upon oral administration, with a terminal elimination half-life of between 7 and 10 hrs. A once-daily dosing of a carvedilol formulation is commercially desirable and would simplify the dosing regimen and enhance patient compliance. Carvedilol exists as a racemate and it contains an α hydroxyl secondary amine, with a pKa of 7.8. It exhibits a predictable aqueous solubility, i.e., above a pH of 9, the solubility is <1 µg/mL and its solubility increases with decreasing pH and reaches a plateau near pH 5; its solubility is about 23 µg/mL at a pH of 7 and about 100 µg/mL at a pH 5. At lower pHs (pH of 1 to 4), solubility is limited by the solubility of the protonated form of carvedilol or its salt form formed *in situ.* The HCl salt form is less soluble than the protonated form itself. Carvedilol is absorbed from the GI tract by transcellular transport. The in vivo absorption decreased within the intestine in the following order: jejunum > ileum >colon. The highest absorption was achieved in the jejunum at a neutral pH. Since the drug dissolution is the rate-limiting factor for absorption of carvedilol in the distal part of the GI tract potentially due to the decrease in solubility, the once-daily dosage form in accordance with one embodiment would comprise at least two bead populations - one IR bead population and another TPR bead population comprising SR coated organic acid cores. Iloperidone is an anti-psychotic agent and Lamotrigine, an anticonvulsant drug, is indicated for the treatment of epilepsy.

In accordance with certain embodiments of the present invention, the solubility enhancing property of organic acid buffers is taken advantage of, and at the same time, the *in situ* formation of acid addition compounds is prevented by having an SR coating membrane between the inner organic acid layer and the weakly basic drug layer. The SR coating membrane thus applied precisely controls the release of the organic acid so as to insure no drug is left behind in the dosage form for lack of solubilizing acid in the TPR bead. In one embodiment, the active core of the dosage form of the present invention may comprise an inert particle coated with an organic acid, an SR coating, drug-layered (IR beads), further barrier or SR coated and/or lag-time coated. The amount of organic acid and the drug-load in the core will depend on the drug, the dose, its pH-dependent solubility, solubility enhancement, and elimination half-life. Those skilled in the art will be able to select an appropriate amount of drug/acid for coating onto the core to achieve the desired BID (twice-daily) or QD (once-daily) dosing regimen. In one embodiment, the inert particle may be a sugar sphere, a cellulose sphere, a silicon dioxide sphere or the like. Alternatively, organic acid crystals with a desired particle size distribution may function as cores, especially for Group C drugs, and in this case, these crystals are membrane coated to program the acid release, which, in accordance with certain embodiments, is synchronized with that of the drug to ensure complete release of the drug prior to depletion of the acid.

In accordance with one aspect of the present invention, the core of the dosage form may comprise an organic acid (e.g., fumaric acid) crystal with a desired mean particle size or an inert particle such as a sugar sphere layered with an organic acid from a polymer binder solution. Organic acid crystals or acid-containing cores are coated with a water-insoluble polymer alone or in combination with a water-soluble or enteric polymer, and the composition and thickness of the SR membrane is optimized such that the acid release is slower than or synchronized with the drug dissolution/release from the bead, thereby ensuring that the acid release is not complete prior to depletion of the drug release. In certain aspects of the invention, the acid-containing cores may be in the form of microgranules or pellets which may be prepared by rotogranulation, high-shear granulation and extrusion-spheronization or compression (as micro-tablets about 1-1.5 mm in diameter) of the organic acid, a polymeric binder and optionally fillers/diluents.

A weakly basic active agent such as carvedilol is layered onto the SR coated fumaric acid-containing beads from a polymeric binder (e.g., povidone) solution and a protective seal-coat comprising a hydrophilic polymer such as Opadry® Clear or Pharmacoat 603 (hypromellose 2910; 3 cps) to form IR beads. In one embodiment, the drug-containing IR beads may be coated twice - an inner barrier coating membrane with a water-insoluble polymer (e.g., ethylcellulose) alone or in combination with a water-soluble polymer and a lag-time coating membrane of a water-insoluble polymer in combination with an enteric polymer to produce TPR beads with a lag-time (release with a delayed-onset) of approximately 1 to 10 hours upon oral administration. The water-insoluble polymer and enteric polymer may be present at a weight ratio of from about 9:1 to about 1:4, preferably at a weight ratio of from about 2:1 to 1:1. The membrane coating typically comprises from about 5% to about 60%, preferably from about 10% to about 50% by weight of the coated beads. In accordance with yet another embodiment, the IR beads may simply be coated with a combination of a water-insoluble polymer and an enteric polymer in the aforementioned amounts.

The unit capsule or conventional tablet dosage form according to the present invention may comprise TPR beads alone or in combination with IR beads while the unit ODT may comprise TPR beads alone or in combination with taste-masked immediate release (IR) beads. IR beads without having a taste-masking membrane will provide rapid release of the weakly basic drug in the gastrointestinal tract within approximately 60 minutes, preferably within 30 minutes following oral administration. If taste-masked, these beads exhibit taste-masking in the buccal cavity and substantially complete release of the weakly basic drug in the gastrointestinal tract within approximately 2 hours, preferably within one hour following oral administration. The TPR beads will release the weakly basic drug over a period of up to approximately 4-20 hours in the gastrointestinal tract after a lag time of about 1-10 hours following oral administration.

The present invention also provides a method for manufacturing a pharmaceutically elegant multiparticulate dosage form having one or more timed, pulsatile release bead populations of one or more weakly basic actives comprising SR-coated organic acid-containing cores, i. e., a well time-controlled, series of pulses so that the active agents and the acid, being deposited in well separated/isolated layers, do not come into contact with each other to form acid-addition compounds until the dosage form comes into contact with a dissolution medium or body fluids following oral ingestion. The dosage form thus produced exhibits composite release profiles of the active agent and the acid that are comparable, more particularly, the acid-release profile is slower than that of the drug so that no undissolved drug is left behind in the dosage form for lack of solubilizing organic acid.

In accordance with one embodiment of the present invention, the method may include the steps of:
a. providing an organic acid-containing core particle (e.g., an organic acid crystal with a desired particle size distribution or a particle comprising an inert particle (e.g., a sugar sphere, a cellulose sphere, a silicon dioxide sphere) layered with an organic acid from a polymeric binder solution);
b. coating the organic acid-containing core particle with an SR coating membrane consisting of a water-insoluble polymer such as EC-10 (ethylcellulose with a mean viscosity of 10 cps) alone or in combination with a water-soluble polymer (e.g., povidone or PEG 400) or an enteric polymer such as hydroxypropyl methylcellulose phthalate (e.g., HP-55);
c. applying a layer of a weakly basic drug onto the SR coated organic acid-containing core particle to form an IR bead;
d. applying a barrier coating membrane onto the IR bead with a solution of a water-insoluble polymer alone or in combination with a water-soluble polymer;
e. applying a lag-time coating membrane onto the SR bead with a solution of a water-insoluble polymer in combination with an enteric at a ratio of about 9:1 to 1:4 to form a timed pulsatile-release drug particle (TPR bead).

In accordance with certain embodiments of the present invention, the method may include the steps of
i. taste-masking IR beads by solvent coacervation with a water-insoluble polymer (e.g., ethylcellulose with a mean viscosity of 100 cps) alone or in combination with a gastrosoluble pore-former (e.g., calcium carbonate) in accordance with the disclosure in the co-pending US Patent Application Ser. No.# 11/213,266 filed Aug. 26,2005 (Publication No. U.S. 2006/0105038 published May 18, 2006) or by fluid-bed coating with a water-insoluble polymer (e.g., ethylcellulose with a mean viscosity of 10 cps) alone or in combination with a gastrosoluble polymer (e.g., Eudragit E100 or EPO) in accordance with the disclosure in the co-pending US Patent Application Ser. No.# 11/248,596 filed Oct. 12, 2005 (Publication No. U.S. 2006/0078614 published April 13, 2006) or a gastrosoluble pore-former (e.g., calcium carbonate) in accordance with the disclosure in the co-pending US Patent Application Ser. No.# 11/256,653 filed Oct. 21, 2005 (Publication No. U.S. 2006/0105039 published May 18, 2006), the contents of the applications set forth in this paragraph are hereby incorporated by reference;
ii. granulating a powder mixture of a sugar alcohol such as mannitol or a saccharide such as lactose and crospovidone, for example, using the disclosure in the co-pending US Patent Application Ser. No.# 10/827,106 filed Apr. 19, 2004 (Publication No. U.S. 2005/0232988 published October 20, 2005), the contents of which are hereby incorporated by reference to produce rapidly-dispersing micro granules;
iii. blending one or more TPR bead populations from step (e) alone or in combination with taste-masked IR beads from step (i), and/or SR beads from step (d) at a desired ratio to provide a desired once-daily plasma profile, rapidly-dispersing microgranules from step (ii) and other pharmaceutically acceptable excipients; and
iv. compressing the blend from step (iii) into orally disintegrating tablets comprising required dose of one or more weakly basic drugs, which would rapidly disintegrate on contact with saliva in the buccal cavity forming a smooth, easy-to-swallow suspension and exhibiting a plasma profile suitable for a twice- or once-daily dosing regimen with reduced incidence of adverse events including non-compliance.

An aqueous or a pharmaceutically acceptable solvent medium may be used for preparing core particles based on coated inert particles. The type of inert binder that is used to bind the water-soluble organic acid or weakly basic drug to the inert particle or to the SR coated acid-containing core is not critical but usually water soluble or alcohol soluble binders, such as polyvinylpyrrolidone (PVP or povidone) or hydroxypropylcellulose may be used. The binder may be used at any concentration capable of being applied to the inert particle. Typically, the binder is used at a concentration of about 0.5 to 10% by weight. The organic acid or the weakly basic drug may be preferably present in this coating formulation in solution or suspension form. The solids content of the drug layering composition may vary depending on the application but typically will vary from about 5 to 30% by weight depending on the viscosity of the coating formulation and/or drug's solubility.

In accordance with other embodiments, the organic acid-containing cores may be prepared by rotogranulation, or by granulation followed by extrusion-spheronization or tableting into micro-tablets. The organic acid, a binder, and optionally other pharmaceutically acceptable excipients (e.g., diluents/fillers) may be blended together in a high-shear granulator, or a fluid bed granulator, such as Glatt GPCG granulator, and granulated to form agglomerates. The wet mass can be extruded and spheronized to produce spherical particles (pellets). The blend comprising acid particles, a binder and optionally a filler/diluent or drug-containing granules can also be compressed into micro-tablets (about 1-1.5 mm in diameter) to produce organic acid-containing pellets. In these embodiments, the acid content could be as high as 95% by weight based on the total weight of the granulated, extruded or compressed core. These acid-containing cores are coated with an SR membrane prior to drug-layering and subsequent coating with functional polymers.

The individual polymeric coatings on the acid-containing cores and IR beads will vary from about 5 to 50% by weight depending on the relative solubility of organic acid to active, nature of the active, composition of the barrier coat, and required lag-time. In one embodiment, the acid cores may be provided with a barrier-coat of a plasticized water-insoluble polymer, such as ethylcellulose (EC-10), at about 5-50% by weight to sustain the acid release over about 5-20 hours. In certain other embodiments, the acid cores may be provided with a barrier-coat of a plasticized ethylcellulose and hydroxypropyl methylcellulose (hypromellose) phthalate (HP-55) at about 10-50% by weight while the IR beads are coated with ethylcellulose (EC-10) at 5-20% by weight to achieve the drug-release synchronized with that of the acid. In yet another embodiment of the present invention, the IR beads may not be provided with any barrier coating, and the outer lag-time coating of EC-10/HP-55/plasticizer at about 45.5/40/14.5 for a weight gain of about 30-50% by weight controls the drug-release following the lag-time. The composition of the membrane layer and the individual weights of the polymers are important factors to be considered for achieving a desired drug/acid-release profile and lag time prior to appreciable drug release.

The drug/acid-release profiles from IR beads, barrier/SR-coated beads and TPR beads may be determined according to the following procedure:

Dissolution testing of IR beads, taste-masked or not, is conducted with a USP Apparatus 1 (baskets at 100 rpm) or Apparatus 2 (paddles at 50 rpm) in 900 mL of 0.1N HCl at 37°C while the dissolution testing of SR and TPR beads is conducted in a USP apparatus using a two-stage dissolution medium (first 2 hours in 700 mL of 0.1N HCl at 37°C followed by dissolution testing at pH = 6.8 obtained by the addition of 200 mL of a pH modifier). Drug/acid-release with time is determined by HPLC on samples pulled at selected intervals.

There are instances wherein the onset of drug release should begin several hours following oral administration to provide adequate plasma concentration to be suitable for a twice- or once-daily dosing regimen, depending on the elimination half-life of the active. In accordance with particular aspects of the invention, drug release may be delayed for up to about 8-10 hours after oral administration.

A single targeted sustained-release profile over several hours after oral administration, with or without an immediate release pulse, is provided in accordance with certain embodiments of the present invention.

An aqueous or a pharmaceutically acceptable solvent medium may be used for preparing organic acid-containing core particles or drug-containing IR Beads by layering the drug onto inert cores such as sugar spheres or onto SR-coated acid-containing cores. The type of inert binder that is used to bind the water-soluble organic acid to the inert particle or the weakly basic drug onto SR-coated acid cores is not critical but usually water-soluble or alcohol and/or acetone-soluble binders are used. Representative examples of binders include, but are not limited to, polyvinylpyrrolidone (PVP), hydroxypropyl methylcellulose (HPMC), hydroxypropylcellulose, carboxyalkylcelluloses, polyethylene oxide, polysaccharides such as dextran, corn starch, which may be dissolved or dispersed in water, alcohol, acetone or mixtures thereof. The binders are typically used at a concentration of from about 0.5 to 10% by weight.

Representative inert particles used to layer the acid or the pharmaceutical active include sugar spheres, cellulose spheres and silicon dioxide spheres with a suitable particle size distribution (e.g. 20-25 mesh sugar spheres for making coated beads for incorporation into a capsule formulation and 60-80 mesh sugar spheres for making coated beads for incorporation into an ODT formulation).

Examples of weakly basic, nitrogen (N)-containing therapeutic agents having a pKa in the range of from about 5 to 14 include, but are not limited to, analgesics, anticonvulsants, antidiabetic agents, anti-infective agents, antineoplastics, antiParkinsonian agents, antirheumatic agents, cardiovascular agents, CNS (central nervous system) stimulants, dopamine receptor agonists, anti-emetics, gastrointestinal agents, psychotherapeutic agents, opioid agonists, opioid antagonists, anti-epileptic drugs, histamine H₂ antagonists, anti-asthmatic agents, and skeletal muscle relaxants.

Representative pharmaceutically acceptable organic acids which enhance the solubility of the pharmaceutical active include citric acid, fumaric acid, malic acid, tartaric acid, succinic acid, oxalic acid, aspartic acid, glutamic acid and the like. The ratio of organic acid to pharmaceutical active typically varies from about 5:1 to 1:10, more particularly from about 3:1 to 1:3 by weight in some embodiments of the present invention.

Representative examples of water-insoluble polymers useful in the invention include ethylcellulose, polyvinyl acetate (for example, Kollicoat SR#30D from BASF), cellulose acetate, cellulose acetate butyrate, neutral copolymers based on ethyl acrylate and methylmethacrylate, copolymers of acrylic and methacrylic acid esters with quaternary ammonium groups such as Eudragit NE, RS and RS30D, RL or RL30D and the like. Representative examples of water-soluble polymers useful in the invention include polyvinylpyrrolidone (PVP), hydroxypropyl methylcellulose (HPMC), hydroxypropylcellulose (HPC), polyethylene glycol, and the like.

Representative examples of enteric polymers useful in the invention include esters of cellulose and its derivatives (cellulose acetate phthalate, hydroxypropyl methylcellulose phthalate, hydroxypropyl methylcellulose acetate succinate), polyvinyl acetate phthalate, pH-sensitive methacrylic acid-methamethacrylate copolymers and shellac. These polymers may be used as a dry powder or an aqueous dispersion. Some commercially available materials that may be used are methacrylic acid copolymers sold under the trademark Eudragit (L100, S100, L30D) manufactured by Rohm Pharma, Cellacefate (cellulose acetate phthalate) from Eastman Chemical Co., Aquateric (cellulose acetate phthalate aqueous dispersion) from FMC Corp. and Aqoat (hydroxypropyl methylcellulose acetate succinate aqueous dispersion) from Shin Etsu K.K.

The enteric, water-insoluble, and water-soluble polymers used in forming the membranes are usually plasticized. Representative examples of plasticizers that may be used to plasticize the membranes include triacetin, tributyl citrate, triethyl citrate, acetyl tri-n-butyl citrate, diethyl phthalate, castor oil, dibutyl sebacate, mono and diacetylated glycerides (commercially available as Myvacet 9-45), and the like or mixtures thereof. The plasticizer, when used, may comprise about 3 to 30 wt.% and more typically about 10 to 25 wt.% based on the polymer. The type of plasticizer and its content depends on the polymer or polymers and nature of the coating system (e.g., aqueous or solvent-based, solution or dispersion-based and the total solids).

In general, it is desirable to prime the surface of the drug-layered particles before applying the barrier-membrane coatings or to separate the different membrane layers by applying a thin hydroxypropyl methylcellulose (HPMC) (e.g., Pharmacoat^{®} 603 or Opadry^{®} Clear) film. While HPMC is typically used, other primers such as hydroxypropylcellulose (HPC) or lower viscosity ethylcellulose can also be used.

The active pharmaceutical ingredients suitable for incorporation into these time-controlled pulsatile release systems include weakly basic active pharmaceutical ingredients, derivatives, or salts thereof, which are nitrogen-containing bioactive moieties having a pKa in the range of from about 5 to 14, a solubility of not more than 200µg/mL at a pH of 6.8, and a ratio of optimal highest dose to solubility at pH 6.8 of not less than about 100. The drug substance can be selected from the group of pharmaceutically acceptable chemical entities with proven pharmacological activity in humans.

Specific examples of weakly basic, nitrogen (N)-containing therapeutic agents include without limitation: olanzapine, a piperazinl derivative indicated for the treatment of schizophrenia, ondansetron or ondansetron hydrochloride, a selective serotonin 5-HT₃ receptor antagonist indicated for the prevention of nausea and vomiting associated with chemotherapy or post-operative surgery, dipyridamole, a dipyrimidine derivative indicated for the prevention of postoperative thromboembolic complications of cardiac valve replacement, carvedilol, a beta-adrenergic blocking agent indicated for the treatment of heart failure of ischemic or cardiomyopathic origin, lamotrigine, a triazine derivative indicated for therapy of epilepsy in adults and pediatric patients, olanzapine or pharmaceutically acceptable salt thereof, a psychotropic agent indicated for the treatment of schizophrenia, quetiapine, a piperazinl derivative indicated for the treatment of bipolar disorders.

The membrane coatings can be applied to the core using any of the coating techniques commonly used in the pharmaceutical industry, but fluid bed coating is particularly useful. The present invention is directed to multi-dose forms, i.e., drug products in the form of multi-particulate dosage forms as hard gelatin capsules, or conventional and orally disintegrating tablets compressed using a rotary tablet press, comprising one or more bead populations for oral administration to provide target PK profiles in patients in need of treatment. The conventional tablets rapidly disperse on entry into the stomach while ODTs rapidly disintegrate on contact with saliva in the oral cavity forming a smooth suspension of coated beads for easy swallowing. One or more coated bead populations may be compressed together with appropriate excipients into tablets (for example, a binder, a diluent/filler, and a disintegrant for conventional tablets while a rapidly dispersing granulation may replace the binder-diluent/filler combination in ODTs). Furthermore, compression into ODTs may be accomplished using a tablet press equipped with an external lubrication system to lubricate punches and dies prior to compression.

The following non-limiting examples illustrate the capsule dosage forms comprising one or more pulses, each with a predetermined delayed-onset and the totality of the *in vitro* drug-release profile or the ensuing *in vivo* plasma concentration profile upon oral administration of the dosage form should mimic the desired profile to achieve maximum therapeutic efficacy to enhance patient compliance and quality of life. Such dosage forms, when administered at the 'right time' or as recommended by the physician, would enable maintaining drug plasma concentration at a level potentially beneficial in minimizing the occurrence of side-effects associated with Cₘₐₓ or Cₘᵢₙ.

**Example 1:**

A. SR Beads of Fumaric Acid

40-80 mesh fumaric acid crystals (3750 g) were charged into a fluid-bed coater, Glatt GPCG 5 equipped with a 9" bottom spray Wurster insert, 10" column length and 16 mm tubing. These acid crystals were coated with a solution (at 6% solids) of 250 g of ethylcellulose (Ethocel Premium 10 cps, referred to hereafter as EC-10) and 166.7 g of polyethylene glycol (PEG 400) at a ratio of 60/40 dissolved in 98/2 acetone/water (6528.3 g) for a weight gain of up to 10% by weight The processing conditions were as follows: atomization air pressure: 2.0 bar; nozzle diameter: 1.00 mm; bottom distribution plate: B; spray/shake interval: 30 s/3 s; product temperature maintained at 35±1°C; inlet air volume: 145-175 cubic feet per minute (cfm) and spray rate increased from about 8 to 30 g/min.

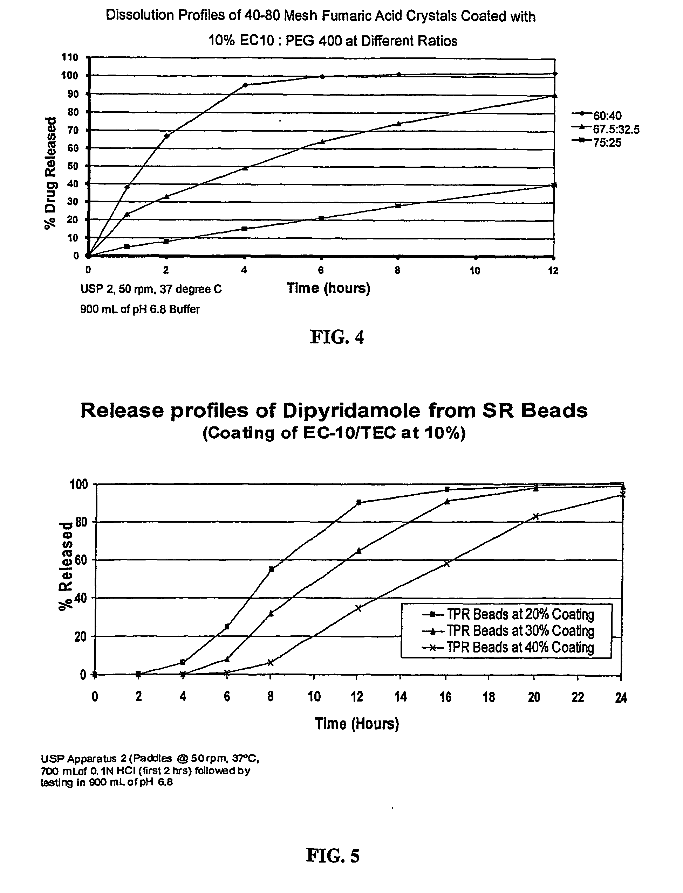

Fumaric acid crystals were also coated as described above using different ratios of ethylcellulose and PEG. More specifically, acid crystals were coated with a solution of EC-10 (Ethocel Premium 10 cps)/PEG 400 at a ratio of either 75/25 or 67.5/32.5 for a weight gain of up to 10% by weight in each case. Fig. 4 shows the release profiles of fumaric acid from fumaric acid crystals coated at different ratios of EC-10/PEG.

B. Barrier-coated Tartaric Acid Crystals

60-100 mesh tartaric acid crystals (900 g) were charged into a fluid-bed coater, Glatt GPCG 1 equipped with a 6" bottom spray Wurster insert, 6" column length, and 1 cm from bottom. These acid crystals were coated with a solution (at 6% solids) of 202.5 g of ethylcellulose (Ethocel Premium 10 cps) and 22.5 g of triethyl citrate (TEC) for a weight gain of 20%. The processing conditions were as follows: atomization air pressure: 1.5 bar; nozzle diameter: 1.00 mm; bottom distribution plate: B; product temperature maintained at 33±1°C; inlet air speed: 4-5 m/s and spray rate increased from about 5 to 8 g/min. Following the coating, the beads were dried in the unit for 10 min to drive off excess of residual solvent The release of tartaric acid was too fast The 20% SR coated crystals released 67% of tartaric acid in an hour when dissolution tested in 0.1N HCl. These coated crystal were coated with EC-10/HP-55/TEC at a ratio of 60/25/15 dissolved in 95/5 acetone/water for a weight gain of 20%. The tartaric acid release at 2 and 4-hr time points were, respectively, 66% and 93% when tested by the two-stage dissolution methodology.

**Example 2:**

A. Fumaric Acid-Containing Cores

Hydroxypropyl cellulose (Klucel LF, 33.3 g) would be slowly added to 90/10 denatured SD 3C 190 proof alcohol/water at 4% solids while stirring rigorously to dissolve and then fumaric acid (300 g) would be slowly added to dissolve. Glatt GPCG 3 equipped with a 6" bottom spray Wurster insert, 8" partition column would be charged with 866.7 g of 25-30 mesh Sugar Spheres. The sugar spheres would be layered with the fumaric acid solution while maintaining the product temperature at about 33-34°C and inlet air velocity at about 3.5-4.5 m/s. The acid cores would be dried in the unit for 10 min to drive off residual solvent/moisture and sieved through 20-30 mesh screens.

B. SR-coated Fumaric acid Cores

The acid cores (1080 g) from above would be coated with a solution (at 7.5% solids) of 108 g of ethylcellulose (EC-10) and 12 g of triethyl citrate (TEC) at a ratio of 90/10 dissolved in 95/5 acetone/water for a weight gain of 10% by weight.

C. Dipyrimadole IR Beads Comprising SR-coated Acid Cores

Dipyrimadole (225 g) would be slowly added to an aqueous solution of polyvinylpyrrolidone Povidone K-29/32 (25 g) to dissolve the drug. SR-coated acid cores would be coated in the Glatt GPCG 3, with the drug solution and the drug-layered beads would be provided with a protective seal-coat of Opadry Clear (about 2% weight gain) to form IR beads with a drug load of 17.29% by weight.

D. Dipyrimadole SR Beads

Dipyrimadole IR beads (1080 g) from above would be barrier-coated (SR coated) by spraying a solution (7.5% solids) of 90/10 EC-10/TEC (triethyl citrate) at 5-10% by weight and dried in the Glatt for 10 minutes to drive off excess residual solvent. The dried beads would be sieved to discard any doubles if formed.

E. Dipyrimadole TPR Beads

Dipyrimadole SR beads (1080 g) with 7% coating from Example 2D would be further coated with a lag-time coating membrane of EC-10/HP-55 (Hypromellose phthalate)/TEC (triethyl citrate) at a ratio of 50/35/15 for a weight gain of about 20%.

The TPR beads would be dried in the Glatt at the same temperature to drive off residual solvent and sieved. Fig. 5 shows the release profiles of fumaric acid from Dipyridamole TPR beads.

**Example 3:**

A. Fumaric Acid-Containing Cores

Hydroxypropyl cellulose (Klucel LF, 20 g) would be slowly added to 90/10 denatured SD 3C 190 proof alcohol/water at 4% solids while stirring rigorously to dissolve and then fumaric acid (200 g) would be slowly added to dissolve. Glatt GPCG 3 would be charged with 780 g of 25-30 mesh sugar spheres. The sugar spheres were layered with the fumaric acid solution as disclosed in Example 1. The acid cores would be dried in the unit for 10 min to drive off residual solvent/moisture and sieved through 20-30 mesh screens.

B. SR-coated Fumaric acid Cores

The acid cores (900 g) from above would be coated with a solution (at 7.5% solids) of 90 g of ethylcellulose (EC-10) and 10 g of triethyl citrate (TEC) at a ratio of 90/10 dissolved in 95/5 acetone/water for a weight gain of 10% by weight.

C. Lamotrigine IR Beads

Lamotrigine (162 g) would be slowly added to an aqueous solution of Klucel LF (13 g) to dissolve the drug. SR-coated acid cores (900 g) from above would be coated in the Glatt GPCG 3 with the drug solution, and the drug-layered beads were provided with a protective seal-coat of Opadry Clear (about 2% weight gain) and dried in the Glatt to produce IR beads.

D. Lamotrigine SR Beads

Lamotrigine IR beads would be barrier-coated by spraying a solution (7.5% solids) of 70/30 EC-10/TEC at 3-5% by weight and dried in the Glatt GPCG 3 at the same temperature for 10 minutes to drive off excess residual solvent. The dried beads would be sieved to discard any doubles if formed.

E. Lamotrigine TPR Beads

Lamotrigine SR beads at 5% coating would be further coated with a lag-time coating membrane of EC-10/HP-55/TEC at a ratio of 42.5/42.5/15 for a weight gain of about 10-15%. The TPR beads would be dried in the Glatt to drive off residual solvent and sieved through 20 mesh sieve.

F. Lamotrigine MR Capsules, 50 mg:

Hard gelatin capsules would be filled with IR beads, SR beads (3%coating) and TPR beads (10% coating) at a ratio of 35/40/25.

**Example 4:**

A. Barrier-coated Tartaric Acid Crystals

60-100 mesh tartaric acid crystals (900 g) would be coated in Glatt GPCG 3 with a solution of fumaric acid (90 g) and 10 g of Klucel LF at a ratio of 90/10 dissolved in denatured SD 3C 190 proof alcohol/water at 4% solids and further coated with EC-10/HP-55/TEC at a ratio of 65/20/15 dissolved in 95/5 acetone/water at 7.5% solids for a gain of 30% by weight as described in the above examples. The coated crystals would be dried and sieved to discard doubles if formed,

B. Lamotrigine IR Beads

Lamotrigine (540 g) would be slowly added to an aaqueous solution of Klucel LF (60 g) to disperse the drug homogeneously. SR-coated acid cores (900 g) from above would be coated in the Glatt GPCG 3 with the drug suspension, and the drug-layered beads would be provided with a protective seal-coat of Opadry Clear for 2% weight gain) and dried in the Glatt to produce IR beads.

C. Lamotrigine SR Beads

Lamotrigine IR beads (800 g) would be SR coated by spraying a solution (7.5% solids) of 85/15 EC-10/TEC for a weight gain of 5-10%. SR beads dried in the Glatt at the same temperature for 10 minutes to drive off excess residual solvent would be sieved to discard any doubles if formed.

D. Lamotrigine TPR Beads

Lamotrigine SR beads would be further coated with a lag-time coating membrane of EC-10/HP-55/TEC at a ratio of 45/40/15 for a weight gain of about 10-20%.

E. Lamotrigine MR Capsules, 50 mg:

Hard gelatin capsules would be filled with IR beads, SR beads (5% or 10% coating) and TPR beads (10% or 20% coating) at a ratio of 35/40/25.

**Example 5:**

A. Fumaric Acid-Containing Cores

Hydroxypropyl cellulose (Klucel LF, 33.3 g) was slowly added to 90/10 190 proof alcohol/water at 4% solids while stirring rigorously to dissolve and then fumaric acid (300 g) was slowly added to dissolve. Glatt GPCG 3 equipped with a 6" bottom spray Wurster insert, 8" partition column is charged with 866.7 g of 60-80 mesh sugar spheres. The sugar spheres were layered with the fumaric acid solution while maintaining the product temperature at about 33-34°C and inlet air velocity at about 3.5-4.5 m/s. The acid cores were dried in the unit for 10 min to drive off residual solvent/moisture and sieved through 40-80 mesh screens.

A. SR-coated Fumaric acid Cores

The acid cores (800 g) from above were coated with a solution (at 7.5% solids) of 180 g of ethylcellulose (EC-10) and 20 g of triethyl citrate (TEC) at a ratio of 90/10 dissolved in 95/5 acetone/water for a weight gain of 10% to 20%.

C. Carvedilol IR Beads

Hydroxypropyl cellulose (Klucel LF, 77.8 g) was slowly added to purified water (at 6% solids) while stirring rigorously to dissolve and Carvedilol (700 g) was slowly added while stirring to disperse the drug homogeneously. SR-coated acid cores (900 g) from above were coated in the Glatt GPCG 3 with the drug dispersion, and the drug-layered beads were provided with a protective seal-coat of Opadry Clear (34.2 g for about 2% weight gain) and dried in the Glatt to produce IR beads.

D. Carvedilol SR Beads

Carvedilol IR beads (1080 g) would be barrier-coated (SR coated) by spraying a solution (7.5% solids) of 90/10 EC-10/TEC at 5% by weight and dried in the Glatt at the same temperature for 10 minutes to drive off excess residual solvent. The dried beads would be sieved to discard any doubles if formed.

E. Carvedilol TPR Beads

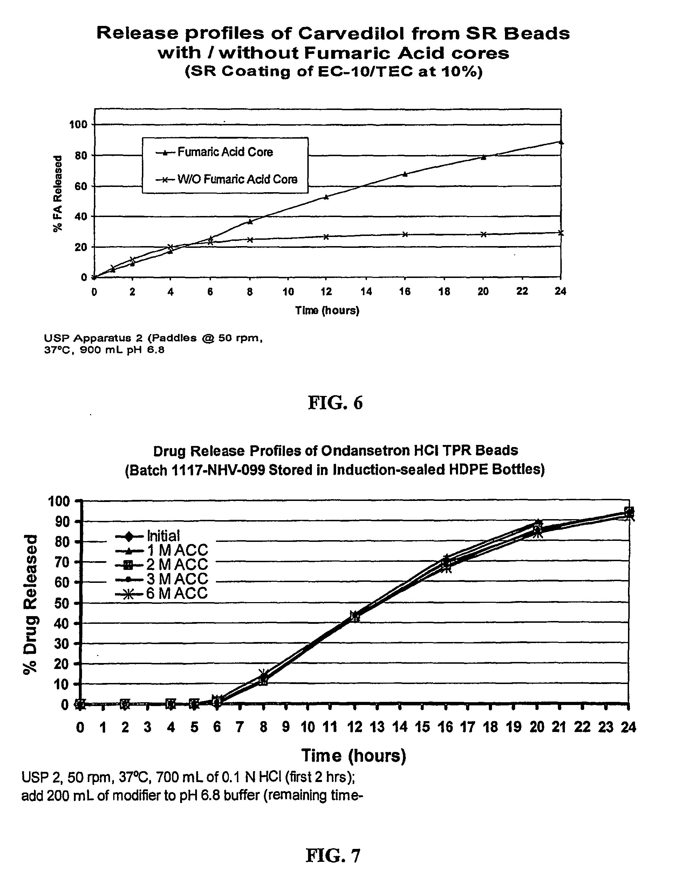

Carvedilol SR beads would be further coated with a lag-time coating membrane of EC-10/HP-55/TEC at a ratio of 50/35/15 for a weight gain of about 10-20% by weight. The TPR beads would be dried in the Glatt to drive off residual solvent and sieved through 20 mesh sieve. Fig. 6 Shows the release profiles of carvedilol TPR beads.

F. Taste-masked IR Beads

The IR beads obtained above would be coated with 50/50 EC-10/Eudragit E100 dissolved in 48.5/24/27.5 acetone/IPA/water for a weight gain of about 10-20% by weight.

G. Rapidly-dispersible Microgranules

The rapidly-dispersible microgranules comprising a sugar alcohol such as mannitol and a disintegrant such as crospovidone were prepared following the procedure disclosed in the co-pending US Patent Application Publication No. U.S. 2005/0232988, published October 20, 2005, the contents of which are hereby incorporated by reference. D-mannitol (152 kg) with an average particle size of approximately 20µm or less (Pearlitol 25 from Roquette, France) were blended with 8 kg of cross-linked povidone (Crospovidone XL-10 from ISP) in a high shear granulator (GMX 600 from Vector) and granulated with purified water (approximately 32 kg) and wet-milled using Comil from Quadro and tray-dried for an LOD (loss on drying) of less than about 0.8%. The dried granules were sieved and oversize material was milled to produce rapidly-dispersible microgranules with an average particle size in the range of approximately 175-300µm.

H. Carvedilol CR ODT

Rapidly-dispersible microgranules would be blended with TPR beads, SR beads, taste-masked IR beads and other pharmaceutical acceptable ingredients, such as flavor, sweetener, and additional disintegrant at a ratio of rapidly-dispersible microgranules to multicoated carvedilol beads of 2:1, in a twin shell V-blender for a sufficient time to get homogeneously distributed blending for compression. Tablets comprising taste-masked beads, SR beads and TPR Beads at a ratio of 35/40/25 as carvedilol would be compressed using a production scale tablet press equipped with an external lubrication system at a mean hardness of about 5-7 kP. Carvedilol MR ODT, 50 mg thus produced would rapidly disintegrate in the oral cavity creating a smooth, easy-to-swallow suspension comprising coated carvedilol beads, which would provide a target profile suitable for a once-daily dosing regimen.

**Example 6:**

A. Fumaric Acid-Containing Cores

Hydroxypropyl cellulose (Klucel LF, 53.6 g) was slowly added to 90/10 190 proof alcohol/water at 4% solids while stirring rigorously to dissolve and then fumaric acid (482.1 g) was slowly added to dissolve. Glatt GPCG 5 equipped with a 9" bottom spray Wurster insert, 10" partition column was charged with 3750 g of 25-30 mesh sugar spheres. The sugar spheres were layered with the fumaric acid solution while maintaining the product temperature at about 33-35°C and a spray rate of 8-60 mL/min. The acid cores were dried in the unit for 10 min to drive off residual solvent/moisture and sieved through 40-80 mesh.

B. SR Coated Fumaric acid Cores

The acid cores (3750 g) from above were coated with a solution (at 7.5% solids) of 177.6 g of ethylcellulose (EC-10) and 19.7 g of triethyl citrate (TEC) at a ratio of 90/10 dissolved in 95/5 acetone/water for a weight gain of 5% by weight following the procedures disclosed above.

C. Ondansetron Hydrochloride Dihydrate IR Beads

Hydroxypropyl cellulose (Klucel LF, 77.8 g) was slowly added to 50/50 190 proof alcohol/water (4247.4 g alcohol+ 4247.4 g water at 5% solids) while stirring rigorously to dissolve and ondansetron HCl (402.8 g) was slowly added while stirring to dissolve the drug. SR coated acid cores (3500 g) were coated in the Glatt GPCG 5 with the drug solution, and the drug-layered beads were provided with a protective seal-coat of Pharmacoat 603 (80.5 g for about 2% weight gain) and dried in the Glatt to produce IR beads (batch size: 4028 g).

D. Ondansetron Hydrochloride SR Beads

Ondansetron Hydrochloride IR beads (3500 g) were barrier-coated (SR coated) by spraying a solution (7.5% solids) of 90/10 EC-10/TEC at 5% by weight and dried in the Glatt at the same temperature for 10 minutes to drive off excess residual solvent. The dried beads woere sieved to discard any doubles if formed.

E. Ondansetron Hydrochloride TPR Beads

Ondansetron Hydrochloride SR beads were further coated with a lag-time coating membrane of EC-10/HP-55/TEC at a ratio of 60.5/25/14.5 for a weight gain of 20% to 45% by weight. The TPR beads were dried in the Glatt to drive off residual solvent and sieved through a 30 mesh sieve. TPR beads packaged in induction sealed HDPE bottles were placed on stability per ICH guidelines. Fig. 7 demonstrates drug release profiles generated on TPR beads on accelerated stability (i.e., at 40°C/75%RH) for up to 6 months. Table 1 demonstrates that the formulation of the present invention is physically and chemically stable.

**Table 1: Stability of Multicoated TPR Beads in induction-sealed HDPE Bottles (Lot# 1117-NHV-099)**

| Time-point (Months) | Description | Average Assay (% of Label Claim) | Moisture (%) | Total Impurities (%) |
|---|---|---|---|---|
| Initial | White to off-white, spherical, free-flowing beads | 100.6 | 1.25 | 0.09 |
| 1 Month at ACC | As Above | 100.6 | 1.02 | 0.14 |
| 2 Month at ACC | As Above | 98.3 | 0.89 | 0.00 |
| 3 Month at ACC | As Above | 97.5 | 1.04 | 0.115 |
| 6 Month at ACC | As Above | 96.9 | 1.4 | 0.109 |

**Example 7 (Comparative):**

A. Carvedilol IR Beads Layered on Sugar Spheres

Hydroxypropyl cellulose (Klucel LF, 77.8 g) was slowly added to 90/10 190 proof alcohol/water (11667 g alcohol+ 1296 g water at 6% solids) while stirring rigorously to dissolve and Carvedilol (700 g) was slowly added while stirring to dissolve the drug. 25-30 mesh Sugar Spheres (900 g) are coated in the Glatt GPCG 3 with the drug solution, and the drug-layered beads were provided with a protective seal-coat of Opadry Clear (34.2 g for about 2% weight gain) and dried in the Glatt to produce IR beads (batch size: 1712 g)).

B. Carvedilol SR Beads

Carvedilol IR beads (800 g) would be barrier-coated (SR coated) by spraying a solution (6% solids) of 80/10 EC-10/TEC at 15% by weight and dried in the Glatt for 10 minutes to drive off excess residual solvent. Pull samples at 5%, 7.5% and 10% coating. The dried beads would be sieved to discard any doubles if formed. Dissolution test 5% and 10% coated SR beads to demonstrate the impact of incorporating an organic acid core.

C. Powder X-ray Diffraction

Powder X-ray diffraction patterns would be generated for fumaric acid, carvedilol, SR coated fumaric acid beads, Carvedilol IR beads, SR beads, and TPR beads of Example 6. The analysis of these X-ray patterns would demonstrate that carvedilol exists in the IR, and TPR beads in the original crystalline state and not as a formate salt.

**Example 8:**

A. Fumaric Acid-Containing Cores

Fumaric acid-containing cores (at a fumaric acid load of 5.4% by weight) were prepared by the procedure described above.

B. SR-coated Fumaric Acid-Containing Cores

The fumaric acid cores (3750 g) from above were coated with a solution of EC-10 and either PEG 400 (B.1) at a ratio of 60/40 or TEC (B.2) at a ratio of 90/10 as the plasticizer, dissolved in 98/2 acetone/water (6% solids) for a weight gain of 10%.

C. Ondansetron Hydrochloride IR Beads

Ondansetron hydrochloride IR beads from B.1 and B.2 above were prepared as disclosed in Example 3 C. The drug-layered beads were provided with a protective seal-coat with Pharmacoat 603 (hypromellose 2910; 3 cps) for a weight gain of 2%.

D. Ondansetron Hydrochloride SR Beads

Ondansetron hydrochloride IR beads (1080 g) were barrier-coated (SR coated) by spraying a solution of EC-10 and either PEG 400 (D.1) at a ratio of 60/40 or TEC (D.2) at a ratio of 90/10 as the plasticizer, dissolved in 98/2 acetone/water (7.5% solids) for a weight gain of 10% and dried in the Glatt at the same temperature for 10 minutes to drive off excess residual solvent. The dried beads were sieved to discard any doubles if formed.

E. Ondansetron Hydrochloride TPR Beads

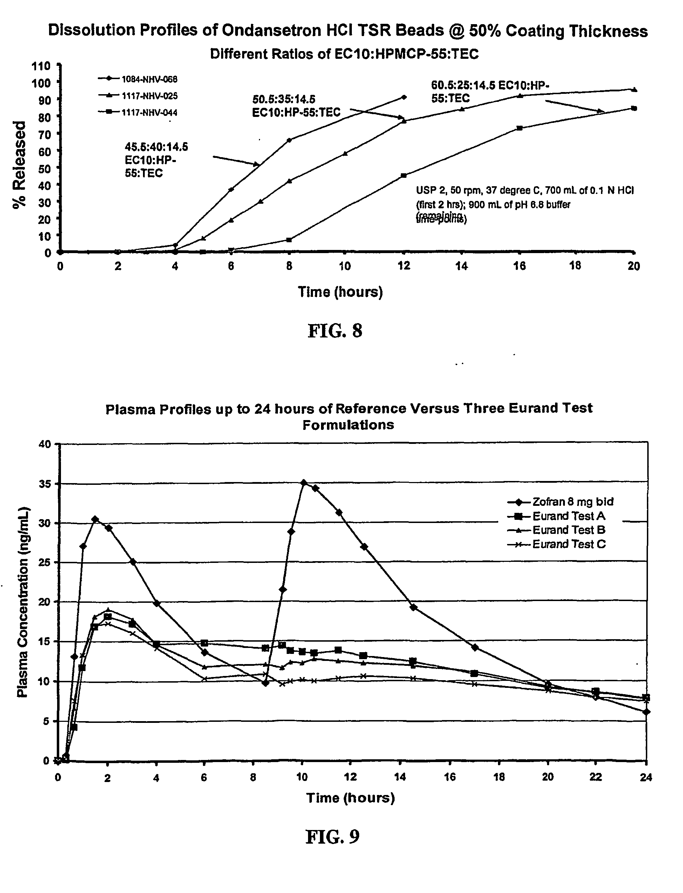

Ondansetron hydrochloride SR beads from D.1 and D.2 above were further coated with a lag-time coating membrane of EC-10/HP-55/TEC at three ratios of 45.5/40/14.5 (E.1 - lot# 1084-066), 50.5/35/14.5 (E.2-lot# 1117-025) and 60.5/25/14.5 (E.3-lot# 1117-044) dissolved in 90/10 acetone/water (7.5% solids) for a gain of up to 50% by weight. The TPR beads were dried in the Glatt to drive off residual solvent and sieved through a 18 mesh sieve. Fig. 8 shows the release profiles for ondansetron hydrochloride from TPR beads coated with EC-10/HP-55/TEC at three different ratios (E.1, E.2 and E.3). More specifically, Fig. 8 shows the release profiles for the following formulations:

(1) TPR beads lot# 1084-066 - The coating of EC-10/HP-55/TEC at a ratio of 45.5/40/14.5 at 50% by weight applied on IR beads coated with 60/40 EC-10/PEG 400 at 10% while IR beads (5% drug layered from 90/10 ondansetron/PVP) comprise fumaric acid cores (4% layered on sugar spheres from acid/Klucel) coated with 60/40 EC-10/PEG 400 at 10%.
(2) TPR beads lot# 1117-025 - The coating of EC-10/HP-55/TEC at a ratio of 50.5/35/14.5 at 50% by weight applied on IR beads coated with 90/10 EC-10/TEC at 10% while IR beads (6% drug layered from 90/10 ondansetron/ Klucel LF) comprise fumaric acid cores (layered on sugar spheres from acid/PVP) coated with 90/10 EC-10/TEC at 10%.
(3) TPR beads lot# 1117-044 - The coating of EC-10/HP-55/TEC at a ratio of 60.5/25/14.5 at 50% by weight applied on IR beads coated with 90/10 EC-10/TEC at 10% while IR beads (6% drug layered from 90/10 ondansetron/Klucel LF) comprise fumaric acid cores (layered on sugar spheres from acid/PVP) coated with 90/10 EC-10/TEC at 10%.

**Example 9:**

A. Proof of Concept Test Formulations

Ondansetron hydrochloride IR beads (PE364EA0001) and TPR beads (lot# PE366EA0001 with a lag-time coating of 30%, lot# PE367EA0001 with a lag-time coating of 45%, and lot# PE368EA0001 with a lag-time coating of 50%) were encapsulated at a ratio of 35% / 65% into hard gelatin capsules to produce MR (modified-release) Capsules, 16 mg (lots# PF380EA0001, lots# PF381EA0001, and lots# PF382EA0001) QD (dosed once-daily) for a pilot bioavailability study in humans in comparison to marketed Zofran^{®} 8 mg (as ondansetron) dosed bid (two times a day).

B. Proof of Concept Human PK Study

A 4-arm crossover pilot POC (proof of concept) study was conducted which included 12 Caucasian male, healthy volunteers aged 18 to 55 years with a wash-out period of 7 days. Each volunteer was dosed with 250 mL of still mineral water a single Test Formulation (16 mg) A (PF380EA0001), B (PF381EA0001), or C (PF382EA0001 of Example 4) at 8 AM or two Zofran^{®} (8 mg) at 8 AM and 4:30 PM after an overnight fasting (at least 12 hrs and lunch was served at11 AM. Blood samples were drawn at 0 (pre-dose), 20 min, 40 min, 1 hr, 1.5 hrs, 2 hrs" 3 hrs, 4 hrs, 6 hrs, 8.5 hrs (before second dose), 9 hrs 10 min, 9.5 hrs, 10 hrs, 10.5s, 11.5 hrs, 12.5 hrs, 14.5 hrs, 17 hrs, 20 hrs, 22 hrs, 24 hrs and 36 hrs. Fig. 9 demonstrates the mean plasma concentration-time profiles achieved. The figure demonstrates that the plasma profiles of Test Formulations A (PE280EA0001), B (PE281EA0001), and C (PE282EA0001) are those characteristic of sustained release formulations, i.e., apparent half-life is significantly longer than that with Zofran. AUC or Cₘₐₓ of Test Formulations does not deviate substantially from that of Zofran (i.e., AUC within ±25% and Cₘₐₓ approximately 70% of Zofran). The actual Cₘₐₓ for Zofran 8 mg was 30 ng/mL in comparison to the predicted 24 ng/mL while the actual Cₘₐₓ for the IR component was about 24 ng/mL when normalized. Approximately 70% of Zofran 8 mg bid (twice-dosed) was absorbed in 24 hrs. Test Formulations A to C exhibited the expected trend post-dosing up to the crossover point at about 15-16 hrs; thereafter, Formula C continued to exhibit a lower plasma concentration-time profile contrary to the predicted behavior.

**Example 10:**

A. SR-coated Tartaric Acid Crystals

60-100 mesh tartaric acid crystals were barrier coated as described in Example 2B.

B. Carvedilol IR Beads (Drug load: 40.9% w/w)

IR beads would be prepared as described in Example 5.

C. Carvedilol SR Beads

The IR beads obtained above would be SR coated with 90/10 EC-10/TEC for a weight gain of 5-10%.

D. Carvedilol TPR Beads

The coated carvedilol SR beads with 5% coating would be coated with a lag-time coating of EC-10/HP-55/TEC at a ratio of 50/35/15 for a weight gain of up to about 30% by weight.

E. Carvedilol CR Capsules, 50 mg

Hard gelatin capsules would be filled with IR beads, SR beads (5% or 10% coating) and TPR beads (30% coating) at a ratio of 35/40/25.

From these demonstrations, it is apparent that the incorporation of an organic acid, as the solubilizer for the weakly basic drugs exhibiting a pH-dependent solubility profile (i.e., showing a decrease in solubility at the intestinal pH 6.8 by about 2 orders of magnitude in comparison to its maximum solubility in the GI fluid) and functional coating of the acid before applying the active pharmaceutical ingredient has significant impact on the lag time, a desired but complete drug release profile prior to depletion of the buffer. Furthermore, the active pharmaceutical ingredient remains in the unaltered form in the solid dosage form until it is released for absorption in the GI tract.
The following numbered paragraphs contain statements of broad combinations of the inventive technical features herein disclosed:-
1. A pharmaceutical multiparticulate dosage form comprising immediate release (IR) beads, one or more populations of sustained-release (SR) beads and/or one or more populations of timed, pulsatile release (TPR) beads of at least one weakly basic drug, wherein the weakly basic drug comprises a pharmaceutically acceptable nitrogen (N)-containing therapeutic agent having a pKa in the range of from about 5 to 14, and a solubility of not more than about 200 µg/mL at pH 6.8 and at least one pharmaceutically acceptable organic acid as a solubilizer wherein the weakly basic therapeutic agent and the organic acid do not come into contact with each other during manufacturing or in storage in the solid state thereby avoiding *in-situ* formation of an acid addition compound and the organic acid is not depleted until completion of the drug release from the dosage form when dissolution tested by United States Pharmacopoeia (USP) dissolution methodology using a two-stage dissolution medium (first 2 hours in 0.1N HCl followed by testing in a buffer at pH 6.8).
2. A pharmaceutical multiparticulate dosage form form in accordance with para 1 wherein the ratio of optimal highest dose for the weakly basic drug to solubility at pH 6.8 is not less than about 100, and at least one pharmaceutically acceptable organic acid solubilizes said weakly basic drug prior to releasing it into a hostile intestinal environment wherein said weakly basic drug is practically insoluble, and said dosage form exhibits target pharmacokinetics profiles at 12-24 hour post-dosing suitable for a twice- or once-daily dosing regimen in patients in need of such a medication.
3. A pharmaceutical multiparticulate dosage form in accordance with para 1 wherein:
   a) said TPR bead comprises an outer lag-time coating comprising a water-insoluble polymer in combination with an enteric polymer applied over said SR bead, said outer coating providing a lag time of from about 2 to about 7 hours before onset of drug release;
   b) said SR bead comprises an SR (barrier) coating surrounding an IR bead, said barrier coating comprising a water-insoluble polymer alone or in combination with a water-soluble pore-forming polymer, said SR membrane providing a sustained-release profile;
   c) said IR bead comprises at least one weakly basic drug applied on a barrier (SR)-coated organic acid core particle;
   d) said barrier-coated organic acid core comprises an inner barrier coating surrounding an organic acid core particle, said inner barrier coating comprises a water-insoluble polymer alone or in combination with a water-soluble polymer or an enteric polymer wherein said inner barrier coating provides a sustained-release profile; and
   e) said organic acid core particle comprises at least one pharmaceutically acceptable organic acid functioning as a solubilizer of said weakly basic drug;
   wherein the ratio of weakly basic drug to organic acid is within a range from about 5:1 to about 1:10.
4. A pharmaceutical multiparticulate dosage form in accordance with para 1 in the form of an orally disintegrating tablet (ODT) further comprising rapidly-dissolving microgranules with an average particle size of not more than 400 µm comprising a disintegrant and a sugar alcohol or a saccharide or a combination thereof, each having an average particle size of not more than about 30 µm wherein said orally disintegrating tablet exhibiting the following properties:
   i. a friability of less than 1% by weight; and
   ii. a disintegration time of about 60 seconds or less on contact with the saliva in the oral cavity forming a smooth suspension comprising multicoated beads.
5. A pharmaceutical multiparticulate dosage form in accordance with para 3 wherein said TPR beads do not include a barrier (SR) coating on said IR beads thereby enabling the release of solubilized drug into a hostile intestinal environment wherein the drug is practically insoluble following oral administration in order to be suitable for a once-daily dosing regimen in patients in need of such a medication.
6. A pharmaceutical multiparticulate dosage form in accordance with para 1 comprising at least an IR bead population, a first TPR bead population and an SR bead population or a second TPR bead population wherein the ratio of IR bead to the first TPR bead to the SR bead or to the second TPR bead populations varies from about 10:90:0 to about 40:10:50.
7. A pharmaceutical multiparticulate dosage form in accordance with para 1 wherein said weakly basic, nitrogen (N)-containing therapeutic agent having a pKa in the range of from about 5 to 14 and a solubility of not more than about 200 µg/mL at pH 6.8, is selected from the group consisting of analgesics, anticonvulsants, antidiabetic agents, anti-infective agents, antineoplastics, antiParkinsonian agents, antirheumatic agents, cardiovascular agents, CNS (central nervous system) stimulants, dopamine receptor agonists, anti-emetics, gastrointestinal agents, psychotherapeutic agents, opioid agonists, opioid antagonists, anti-epileptic drugs, histamine H₂ antagonists, anti-asthmatic agents, and skeletal muscle relaxants.
8. A pharmaceutical multiparticulate dosage form of para 7 wherein said weakly basic, nitrogen (N)-containing therapeutic agent is selected from the group consisting of olanzapine, ondansetron, ondansetron hydrochloride, dipyridamole, carvedilol, lamotrigine, olanzapine, quetiapine, pharmaceutically acceptable salts thereof and combinations thereof.
9. A pharmaceutical multiparticulate dosage form in accordance with para 1 wherein the organic acid is selected from the group consisting of citric acid, fumaric acid, malic acid, maleic acid, tartaric acid, succinic acid, oxalic acid, aspartic acid, glutamic acid and mixtures thereof.
10. A pharmaceutical multiparticulate dosage form in accordance with para 1 wherein the ratio of weakly basic drug to organic acid varies from about 5:1 to 1:10 by weight to provide target pharmacokinetic profiles suitable for a once-daily dosing regimen.
11. A pharmaceutical multiparticulate dosage form in accordance with para 3, wherein said organic acid core particle comprises:
   i. an organic acid crystal;
   ii. inert particle coated with an organic acid and a polymer binder; or
   iii. a pellet or a micro-tablet containing the organic acid, a polymer binder and a diluent/filler, prepared by rotogranulation, granulation-extrusion-spheronization or granulation-compression.
12. A pharmaceutical multiparticulate dosage form in accordance with para 11 wherein said IR bead comprises a drug layer comprising a polymeric binder at a drug to binder ratio of from about 85:15 to about 99:1 and said polymeric binder is selected from the group consisting of polyvinylpyrrolidone, methylcellulose, hydroxypropylcellulose, hydroxypropyl methylcellulose, com starch, pre-gelatinized starch and mixtures thereof.
13. A pharmaceutical multiparticulate dosage form in accordance with para 3 wherein said particle core is provided with a barrier (SR) coating comprising a water-insoluble polymer alone or in combination with a water soluble polymer at a ratio of from about 9:1 to 5:5 wherein said barrier coating is applied for a weight gain of from about 1.5% to 20% by weight based on the weight of the coated bead.
14. A pharmaceutical multiparticulate dosage form in accordance with para 11 I wherein said particle barrier coating comprises a water-insoluble polymer selected from the group consisting of ethylcellulose, cellulose acetate, cellulose acetate butyrate, polyvinyl acetate, neutral methacrylic acid-methylmethacrylate copolymers, and mixtures thereof.
15. A pharmaceutical multiparticulate dosage form in accordance with para 11 wherein said particle core is provided with a barrier coating comprising a water-insoluble polymer alone or in combination with a water soluble polymer selected from the group consisting of methylcellulose, hydroxypropyl methylcellulose, hydroxypropylcellulose, polyvinyl pyrrolidone and polyethylene glycol and mixtures thereof.
16. A pharmaceutical multiparticulate dosage form in accordance with para 3 wherein said lag-time coating comprises a water-insoluble polymer in combination with an enteric polymer at a ratio of from about 9:1 to 1:3, respectively, for a weight gain of from about 10% to 60% by weight based on the weight of the TPR bead.
17. A pharmaceutical multiparticulate dosage form in accordance with para 14 wherein said lag-time coating comprises a water-insoluble polymer in combination with an enteric polymer selected from the group consisting of cellulose acetate phthalate, hydroxypropyl methylcellulose phthalate, hydroxypropyl methylcellulose succinate, polyvinyl acetate phthalate, pH-sensitive methacrylic acid-methylmethacrylate copolymers, shellac, derivatives thereof, and mixtures thereof.
18. A pharmaceutical multiparticulate dosage form in accordance with para 3 wherein at least one of the inner barrier coatings and the outer lag-time coating comprises a plasticizer selected from the group consisting of triacetin, tributyl citrate, tri-ethyl citrate, acetyl tri-n-butyl citrate, diethyl phthalate, dibutyl sebacate, polyethylene glycol, polypropylene glycol, castor oil, acetylated mono- and di-glycerides and mixtures thereof.
19. A pharmaceutical multiparticulate dosage form of para 3 wherein said IR beads provide a loading dose by releasing not less than about 50% of the active contained in said IR beads within the first hour after oral administration of the dosage form.
20. A pharmaceutical multiparticulate dosage form of para 3 wherein said IR bead, if incorporated as an IR portion of the dosage form, comprises said weakly basic drug and a polymer binder layered on an inert core.
21. A pharmaceutical multiparticulate dosage form in accordance with para 1 wherein said weakly basic drug comprises carvedilol or pharmaceutically acceptable salt thereof, and each TPR bead population comprises sustained-release coated tartaric acid cores coated with a lag-time coating of water-insoluble ethylcellulose and enteric hydroxypropyl methylcellulose phthalate at a ratio of from about 9:1 to about 1:3 for a weight gain of up to 50%, exhibiting upon oral administration of the dosage form a pre-determined lag-time followed by differing release characteristics.
22. A pharmaceutical multiparticulate dosage form in accordance with para 1 wherein said weakly basic drug comprises ondansetron or pharmaceutically acceptable salt thereof, and each TPR bead population comprises sustained-release coated fumaric acid cores coated with a lag-time coating of water-insoluble ethylcellulose and enteric hydroxypropyl methylcellulose phthalate at a ratio of from about 9:1 to about 1:3 for a weight gain of up to 50%, exhibiting upon oral administration of the dosage form a pre-determined lag-time followed by differing release characteristics.
23. A pharmaceutical multiparticulate dosage form in accordance with para 1 wherein said orally disintegrating tablet comprises a taste-masked IR dead population, an SR bead population, and /or one or two TPR bead populations of a weakly basic, nitrogen (N)-containing therapeutic agent having a pKa in the range of from about 5 to 14 or pharmaceutically acceptable salt thereof, wherein each SR or TPR bead population comprising sustained-release coated fumaric acid cores, rapidly disintegrates in the oral cavity creating a smooth, easy-to-swallow suspension of multi-coated beads to provide target pharmacokinetics profiles suitable for a once-or twice-daily dosing regimen in patients in need of such a medication.
24. A method for the preparation of a multiparticulate dosage form comprising a weakly basic, nitrogen (N)-containing therapeutic agent having a pKa in the range of from about 5 to 14 and a solubility of not more than about 200 µg/mL at pH 6.8, and at least one pharmaceutically acceptable organic acid as a solubilizer comprising:
   a. preparing organic acid cores;
   b. preparing barrier-coated organic acid cores by coating the organic acid cores with a barrier-coating comprising a polymer, more particularly comprising a water-insoluble polymer alone or in combination with a water-soluble polymer or an enteric polymer at a ratio of from about 95/5 to about 50/50 for a weight gain of up to about 20%, to provide a sustained-release profile;
   c. preparing IR (immediate-release) beads by layering the one or more weakly basic or pharmaceutically acceptable salts thereof from a polymer binder solution onto the barrier-coated organic acid cores and optionally applying a protective seal-coat with a water-soluble polymer;
   d. preparing SR beads by applying a barrier (SR) coating of a water-insoluble polymer alone or in combination with a water-soluble polymer at a ratio of from about 95:5 to about 50:50 for a weight gain of from about 1.5% to 20% by dry weight of the coated bead;
   e. preparing TPR beads by applying an outer lag-time coating comprising a water-insoluble polymer in combination with an enteric polymer at a ratio of from about 9:1 to 1:3 for a weight gain of from about 10% to 60% by weight of the coated bead; and
   f. filling into a gelatin capsule or compressing into a conventional tablet or an orally disintegrating tablet a mixture of IR beads, SR beads and/or one or more TPR bead populations at appropriate amounts to achieve target pharmacokinetics profiles in order to be suitable for a once-daily dosing regimen in patients in need of such a medication. pharmacokinetics profiles in order to be suitable for a twice- or once-daily dosing regimen in patients in need of such a medication.
25. A method in accordance with para 22, wherein each of said organic acid-layering, SR-coating, drug-layering and outer lag-time coating is applied from a solution in a pharmaceutically acceptable solvent system or from an aqueous dispersion.
26. A method in accordance with para 22 further comprising the following steps:
   i. optionally taste-masking drug-containing beads either by solvent coacervation or by fluid-bed coating;
   ii. optionally providing a compressible coating on IR beads, SR beads and/or TPR beads with a plasticized polymer to eliminate/minimize membrane fracture during compression;
   iii. granulating a sugar alcohol or a saccharide, or a combination thereof, and a disintegrant, each having an average particle size of not more than about 30 µm to produce rapidly dispersing microgranules with an average particle size of not more than about 400 µm;
   iv. blending the multi-coated beads with the rapidly dispersing microgranules at a ratio of multi-coated beads to microgranules from about 1:6 to about 1:2; and
   v. compressing the blend into orally disintegrating tablets on a conventional rotary tablet press.
27. A method in accordance with para 24, wherein said step of compressing may comprise utilizing a conventional rotary tablet press equipped with an external lubrication system to lubricate the dies and punches prior to compression.
28. method of para 22 wherein the dosage form comprises therapeutically effective amounts of IR bead population, SR bead population and/or one or more TPR bead populations of a weakly basic, nitrogen (N)-containing therapeutic agent, each multicoated bead population exhibiting differing release characteristics following a pre-determined lag-time.

## Claims

1. A pharmaceutical multiparticulate dosage form comprising one or more populations of timed, pulsatile release (TPR) beads of at least one weakly basic drug,
wherein the weakly basic drug comprises a pharmaceutically acceptable nitrogen (N)-containing therapeutic agent, or a pharmaceutically acceptable salt thereof, having a pKa in the range of from about 5 to 14, and a solubility of not more than about 200 µg/mL at pH 6.8;
wherein the TPR beads comprise organic acid core particles comprising at least one pharmaceutically acceptable organic acid, which are coated with an inner barrier coating comprising a water-insoluble polymer alone or in combination with a water-soluble polymer or an enteric polymer, on which the weakly basic drug is layered and further coated with a lag-time membrane,
such that the inner barrier coating is disposed between the organic acid layer and the weakly basic drug layer.

2. The pharmaceutical multiparticulate dosage form of claim 1, wherein the weakly basic drug has a ratio of optimal highest dose to solubility at pH 6.8 of not less than about 100, and
wherein said dosage form optionally exhibits a pharmacokinetic profile at 24 hours post-dosing suitable for a once-daily dosing regimen for patients in need thereof.

3. The pharmaceutical multiparticulate dosage form of claim 1, wherein said TPR beads comprise an outer lag-time coating comprising a water-insoluble polymer in combination with an enteric polymer, said outer lag-time coating providing a lag time of from about 2 to about 7 hours before onset of release of the weakly basic drug;
wherein said outer lag-time coating is disposed over a sustained-release (SR) coating comprising a water-insoluble polymer alone or in combination with a water soluble polymer,
wherein said SR coating is disposed over the weakly basic drug layer;
wherein the weakly basic drug layer is disposed over the inner barrier-coating, which is disposed over the organic acid containing cores.

4. The pharmaceutical multiparticulate dosage form of claim 1, in the form of an orally disintegrating tablet (ODT), wherein, optionally,
(a) said ODT further comprises rapidly-dispersing microgranules which comprise a disintegrant and a sugar alcohol or a saccharide or a combination thereof, and each of the disintegrant and sugar alcohol or saccharide have an average particle size of not more than about 30 µm,
(b) said microgranules optionally have an average particle size of not more than 400 µm, and
(c) said ODT optionally has a friability of less than 1% by weight, and a disintegration time of about 60 seconds or less on contact with saliva in the oral cavity.

5. The pharmaceutical multiparticulate dosage form of claim 1, wherein said TPR beads comprise:
an outer lag-time coating comprising a water-insoluble polymer in combination with an enteric polymer; said outer lag-time coating providing a lag time of from about 2 to about 7 hours before onset of release of the weakly basic drug, wherein said outer lag-time coating is disposed over the weakly basic drug layer, which is disposed over the inner barrier coating, which is disposed over the organic acid containing core particles.

6. The pharmaceutical multiparticulate dosage form of any preceding claim wherein
(a) said weakly basic drug is selected from the group consisting of analgesics, anticonvulsants, antidiabetic agents, anti-infective agents, antineoplastics, antiParkinsonian agents, antirheumatic agents, cardiovascular agents, CNS (central nervous system) stimulants, dopamine receptor agonists, anti-emetics, gastrointestinal agents, psychotherapeutic agents, opioid agonists, opioid antagonists, anti-epileptic drugs, histamine H2 antagonists, anti-asthmatic agents, and skeletal muscle relaxants, wherein said weakly basic drug is optionally selected from the group consisting of olanzapine, ondansetron, ondansetron hydrochloride, dipyridamole, carvedilol, lamotrigine, olanzapine, quetiapine, pharmaceutically acceptable salts thereof, and combinations thereof
(b) the organic acid is selected from the group consisting of citric acid, fumaric acid, malic acid, maleic acid, tartaric acid, succinic acid, oxalic acid, aspartic acid, glutamic acid and mixtures thereof
(c) the ratio of the weakly basic drug to the organic acid varies from about 5:1 1 to 1:10 by weight, or
(d) the organic acid is not depleted from said dosage form until completion of the release of the weakly basic drug from the dosage form when dissolution tested by United States Pharmacopoeia (USP) dissolution methodology using a two-stage dissolution medium (first 2 hours in 0.1N HCl followed by testing in a buffer at pH 6.8)
(e) the lag-time coating comprises ethyl cellulose and hydroxypropylmethyl cellulose phthalate.

7. The pharmaceutical multiparticulate dosage form of claim 1 or claim 3, wherein said organic acid core particles are in the form of:
i. an organic acid crystal;
ii. inert particle coated with an organic acid and a polymer binder; or
iii. a pellet or a micro-tablet comprising the organic acid, a polymer binder and a diluent/filler, and
optionally wherein the dosage form is as claimed in claim 3 and the drug layer on the organic acid core particles comprises the weakly basic drug and a polymeric binder at a drug to binder ratio of from about 85:15 to about 99:1; and said polymeric binder is selected from the group consisting of polyvinylpyrrolidone, methylcellulose, hydroxypropylcellulose, hydroxypropyl methylcellulose, corn starch, pre-gelatinized starch and mixtures thereof.

8. The pharmaceutical multiparticulate dosage form of claim 3, wherein
(a) the inner barrier comprises a water-insoluble polymer alone, or a water-insoluble polymer in combination with a water soluble polymer at a ratio of from about 9:1 to 5:5; and wherein said inner barrier coating is applied for a weight gain of from about 1.5% to 20% by weight based on the weight of the SR-coated organic acid core particles, or
(b) said lag-time coating comprises a water-insoluble polymer in combination with an enteric polymer at a ratio of from about 9:1 to 1:3, respectively, for a weight gain of from about 10% to 60% by weight based on the dry weight of the TPR bead.

9. The pharmaceutical multiparticulate dosage form of any preceding claim, wherein
(a) said water-insoluble polymer is selected from the group consisting of ethylcellulose, cellulose acetate, cellulose acetate butyrate, polyvinyl acetate, neutral methacrylic acid-methylmethacrylate copolymers, and mixtures thereof
(b) said water soluble polymer is selected from the group consisting of methylcellulose, hydroxypropyl methylcellulose, hydroxypropylcellulose, polyvinyl pyrrolidone and polyethylene glycol and mixtures thereof,
(c) the enteric polymer is selected from the group consisting of cellulose acetate phthalate, hydroxypropyl methylcellulose phthalate, hydroxypropyl methylcellulose succinate, polyvinyl acetate phthalate, pH-sensitive methacrylic acid-methylmethacrylate copolymers, shellac, derivatives thereof, and mixtures thereof, and/or
(d) at least one of the SR coating, the inner barrier coating and the outer lag-time coating further comprises a plasticizer selected from the group consisting of triacetin, tributyl citrate, tri-ethyl citrate, acetyl tri-n-butyl citrate, diethyl phthalate, dibutyl sebacate, polyethylene glycol, polypropylene glycol, castor oil, acetylated mono- and di-glycerides and mixtures thereof.

10. The pharmaceutical multiparticulate dosage form of claim 1, wherein
(a) said weakly basic drug comprises carvedilol or a pharmaceutically acceptable salt thereof and the organic acid is tartaric acid; wherein the lag-time coating comprises ethylcellulose and hydroxypropyl methylcellulose phthalate at a ratio of from about 9:1 to about 1:3 for a weight gain of up to 50%, and each population of TPR beads exhibits upon oral administration of the dosage form a pre-determined lag-time and differing release characteristics or
(b) said weakly basic drug comprises ondansetron or a pharmaceutically acceptable salt thereof and the organic acid is fumaric acid; wherein the lag-time coating comprises ethylcellulose and hydroxypropyl methylcellulose phthalate at a ratio of from about 9:1 to about 1:3 for a weight gain of 5% to 60%, and each population of TPR beads exhibits upon oral administration of the dosage form a pre-determined lag-time and differing release characteristics.

11. A method of producing a pharmaceutical multiparticulate dosage form comprising one or more populations of timed, pulsatile release (TPR) beads and/or one or more populations of sustained-release (SR) beads of at least one weakly basic drug and at least one pharmaceutically acceptable organic acid, wherein the weakly basic drug is a pharmaceutically acceptable nitrogen (N)-containing drug, or a pharmaceutically acceptable salt thereof, having a pKa in the range of from about 5 to 14, and a solubility of not more than about 200 µg/mL at pH 6.8, the method comprising:
a) preparing organic acid-containing core particles;
b) preparing inner barrier-coated organic acid core particles by coating the acid-containing core particles with a sustained-release membrane comprising a water-insoluble polymer or a water-insoluble polymer in combination with a pore-forming water-soluble or enteric polymer;
c) preparing immediate release (IR) beads by coating a solution comprising the weakly basic drug, and optionally applying a protective seal-coat, onto the inner barrier-coated acid-containing core particles; and
d) preparing SR beads by applying an SR coating membrane comprising a water-insoluble polymer or a water-insoluble polymer in combination with a water-soluble polymer, on the IR bead prepared according to step (c); and/or
e) preparing TPR beads, by applying a lag-time coating membrane comprising a combination of a water-insoluble polymer and an enteric polymer to form a TPR bead on an SR-coated bead prepared according to step (d) or directly on an IR bead prepared according to step (c).

12. A method according to claim 11 comprising:
a. preparing organic acid-containing particles comprising at least one pharmaceutically acceptable organic acid;
b. preparing inner barrier coated organic acid-containing particles by coating the organic acid-containing particles with an inner barrier coating comprising a water-insoluble polymer alone, or a water-insoluble polymer in combination with a water-soluble polymer or an enteric polymer at a ratio of from about 95/5 to about 50/50 for a weight gain of up to about 20%;
c. preparing IR beads by coating a solution comprising the weakly basic drug, or a pharmaceutically acceptable salt thereof, and a polymer binder, and optionally applying a protective seal-coat comprising a water-soluble polymer, onto the inner barrier-coated organic acid-containing particles;
d. preparing SR beads by applying a barrier (SR) coating of a water-insoluble polymer alone, or a water-insoluble polymer in combination with a water-soluble polymer at a ratio of from about 95:5 to about 50:50 onto the organic acid core particles for a weight gain of from about 1.5% to 20% by dry weight of the coated SR beads;
e. preparing TPR beads by applying an outer lag-time coating comprising a water-insoluble polymer in combination with an enteric polymer to the SR beads at a ratio of from about 9:1 to 1:3 for a weight gain of from about 10% to 60% of the total dry weight of the TPR beads; and
f. filling into a capsule or compressing into a tablet or an orally disintegrating tablet, one or more TPR and/or SR bead populations in amounts sufficient to provide a pharmacokinetic profile suitable for a once-daily dosing regimen in patients in need of such a medication
optionally wherein the pharmaceutical multiparticulate dosage form comprises therapeutically effective amounts of two or more TPR bead populations, wherein each of two or more TPR bead populations exhibit differing release characteristics and a pre-determined lag-time.

13. The method of claim 12 wherein the method is a method of preparing the multiparticulate dosage form of claim 1 and step (f) comprises:
filling into a capsule or compressing into a tablet or an orally disintegrating tablet, one or more TPR bead populations in amounts sufficient to provide a pharmacokinetic profile suitable for a once-daily dosing regimen in patients in need of such a medication, optionally in combination with IR beads.

14. The method of any one of claims 11 to 13, wherein each of said coating or applying steps comprises coating or applying from a solution in a pharmaceutically acceptable solvent system or from an aqueous dispersion.

15. The method of any one of claims 11 to 13, wherein step (f) includes compressing the beads into an orally disintegrating tablet, optionally compressing the beads on a tablet press equipped with an external lubrication system to lubricate the dies and punches prior to compression
and wherein said method further comprises:
g. optionally taste-masking one or more bead populations by solvent coacervation or by fluid-bed coating prior to compressing
h. optionally providing a compressible coating on said one or more TPR and/or SR beads, wherein said compressible coating comprises a plasticized polymer, and whereby coating fracture is minimized during compression, and
i. granulating a powder mixture of a sugar alcohol or a saccharide, or a combination thereof, and a disintegrant, each having an average particle size of not more than about 30 µm to produce rapidly dispersing microgranules;
j. blending the bead populations with the rapidly dispersing microgranules; and
k. compressing the blend of step (iv) into orally disintegrating tablets.

16. Use of an aqueous or pharmaceutically acceptable solvent medium for preparing organic acid-containing core particles for drug layering by layering an acid onto sugar spheres or IR beads and by layering drug onto acid-containing cores or directly onto sugar spheres from an appropriate polymer binder solution in fluid-bed equipment.
